# EUROPEAN PATENT APPLICATION

(11) **EP 2 987 501 A1**
(43) Date of publication of application: **24.02.2016**
(21) Application number: 15181996.8
(22) Date of filing: 14.10.2011
(51) Int. Cl.: A61K 39/36, A61K 39/00, A61K 38/38, A61P 37/00

(54) **SUPPRESSION OF A HYPERSENSITIVITY IMMUNE RESPONSE WITH UNRELATED ANTIGEN DERIVED FROM ALLERGEN SOURCE MATERIAL**

(30) Priority: 15.10.2010 US 393483 P; 15.10.2010 EP 10187745; 11.03.2011 EP 11157869
(62) Divisional of application: 11771114.3
(71) Applicant: Alk-Abelló A/S, 2970 Hørsholm (DK)
(72) Inventor: Brimnes, Jens, 2860 Soborg (DK); Lund, Kaare, 2830 Virum (DK)
(74) Representative: Inspicos P/S

(57) **Abstract**

The present invention relates to the treatment of a hypersensitivity immune response, such as allergic rhinitis or asthma, via bystander suppression by use of an antigen unrelated to the allergen triggering the hypersensitivity immune responsein an individual to be treated, wherein the antigen is obtainable from the source material comprising the "triggering" allergen.

## Description

### Technical Field

The present invention is within the field of immunotherapy, treatment of hypersensitivity immune responses and bystander suppression.

### Background

Allergen-specific immunotherapy (SIT) was introduced into clinical medicine almost a century ago for the treatment of type I hypersensitivity immune responses and SIT is currently the only treatment leading to prolonged tolerance against allergens. In SIT, the specific allergen or a cross-reacting allergen thereof is repeatedly administered to the individual, usually either by subcutaneous administration or by sublingual administration, during a longer period, usually more than one year. Typically, the patient experiences lower symptom scores on re-exposure to the allergen(s) after some weeks or months treatment (Allergens and Allergen Immunotherapy 4th Ed, 2008, Ed by R Lockey and D Ledford, Informa healthcare).

One challenge encountered with SIT is that the allergen needs to be identified and that the individual undergoing SIT has significant elevated risk of getting severe systemic adverse reactions like anaphylaxis as well as local adverse reactions like swelling and itching. Therefore, there is a need to improve the treatment of a hypersensitivity immune response.

About 20 years ago, Miller et al (1991) demonstrated the principle of providing peripheral tolerance to an antigen (MBP) (myelin basic protein) in naïve rats not yet sensitized to MBP antigen by treatment of the naïve rats by intra-gastric administration of OVA (an antigen unrelated to MBP) before the rats were exposed to MBP for the first time. Miller et al (1991) found that the rats were only protected from developing EAE, if the rats were co-exposed to the unrelated antigen (OVA) along with being exposed to the MBP for the first time.

Later on several researchers have investigated bystander suppression to an immune response by administering an antigen unrelated to the antigen triggering the immune response.

For example, Dahlman-Hoglund et al (1995) have investigated the suppression of an immune response in naïve rats subsequent to feeding the naïve rats orally with a first antigen prior to challenging the naïve rat to a second antigen so as to trigger an inadvertent immune response. The investigators found that the immune response due to the second antigen was suppressed only where the rats were exposed to a mixture containing both the first antigen and the second antigen at the time of triggering the inadvertent immune response. In contrast, suppression of the immune response was not achieved when the first and second antigens were administered at two separate body parts of the rat.

The publication of Millington et al (2004) relates to feeding mice with a soluble protein (OVA) so as to induce bystander suppression to an unrelated antigen.

Oliveira CR et al (2005) have suggested bystander effect of a non-related antigen as an interesting mechanism to control sensitization to a new allergen in individuals already sensitized to one allergen. The authors have indicated that per-oral pretreatment with OVA leads to bystander down regulation of specific antibodies towards the allergen, but only when OVA is present at the subsequent immunization.

The publication of Wang Lifang et al (2009) relates to antigen-driven bystander effect that accelerates epicutaneous sensitization with a new protein allergen. The publication of Brimnes et al (2009) relates to the investigation of sublingual immunotherapy in naïve mice. The publication of Kildsgaard et al (2005) relates to the investigation of sublingual immunotherapy in sensitized mice. The publication of Weiner et al (1997) relates to oral tolerance and treatment of autoimmune diseases. The publication of Rask et al (2010) relate to the investigation of sublingual immunotherapy in a mouse model of allergic inflammation. The publication of Brimnes et al (2007) relates to the investigation of sublingual immunotherapy in a mouse model of rhinitis. The publication of Kildsgaard et al (2007) relates to the investigation of sublingual immunotherapy in sensitized mice.

The international patent application WO 2004/082710 relates to a product comprising i) a modulator of the Notch signalling pathway; and ii) an allergen or allergen bystander antigen or antigenic determinant thereof, or a polynucleotide coding for an allergen or allergen bystander antigen or antigenic determinant thereof; as a combined preparation for simultaneous, contemporaneous, separate or sequential use for modulation of the immune system.

### Summary of the Invention

In contrast to conventional allergen specific immunotherapy where the individual is treated with the specific allergen (incl. a mixture of allergens or cross-reacting allergens thereof) triggering the hypersensitivity immune response, the present invention relates to the treatment, including prophylactic treatment, of a hypersensitivity immune response in an individual with an antigen unrelated to the allergen triggering a hypersensitivity immune response in the individual. That is to say that the present invention also relates to bystander suppression of a hypersensitivity immune response.

The present inventors have provided evidence that a hypersensitivity immune response triggered by exemplary antigens in mice could be significantly reduced if the mice where pre-treated with another antigen unrelated to the "triggering" antigen. Interestingly, the alleviation of the hypersensitivity immune response was also reflected in clinically relevant readouts of an allergic immune response, e.g. reduction in number of sneezes or reduction in airway hyperresponsiveness. Even more interestingly, the alleviation of the hypersensitivity immune response could be demonstrated in mice pre-sensitized to the "triggering" antigen as well as in mice not sensitized to the "triggering" antigen. The inventors have also found that the immune response was only reduceable, if the unrelated antigen is available together with the "triggering" antigen at the same time as the mice are challenged/ exposed to the "triggering" antigen.

Thus, it may be understood that the present treatment of an immune response with an antigen unrelated to the "triggering" antigen only is workable, if the target organ of the "triggering" antigen is also exposed to the unrelated antigen at the same time as the "triggering" antigen. For example, it might be envisaged that the treatment with an unrelated antigen of an individual suffering from a hypersensitivity immune response due to exposure to an inhalation allergen is only workable, if the unrelated antigen is also exposed to the respiratory tract of the individual.

As recognized by the present inventors, it is a highly challenging task to ensure co-availability / co-exposure of both the antigen triggering the inadvertent immune response and the unrelated antigen at the organ target for the natural exposure of a "triggering" antigen. At least, it is envisaged that where the target organ is the respiratory tract, it is a challenge to ensure co-exposure of a pollen allergen and an unrelated antigen, unless the individual administers the unrelated antigen to the respiratory tract concomitantly during exposure to the airborne allergen material (e.g. pollen).

The present inventors have now found that co-exposure might be provided by selecting an unrelated antigen that is naturally exposed to the target organ along with the antigen triggering the hypersensitivity immune response in the individual. As such the unrelated antigen inherently reaches the same target organ as the "triggering" antigen upon the natural exposure to the "triggering" antigen. For example, the present inventors have found that the unrelated antigen might be selected among antigens present in a source material also comprising the antigen triggering a hypersensitivity immune response in an individual, such as be selected among antigens present in pollen, animal dander or a dietary product comprising an antigen (i.e. allergen) capable of triggering a hypersensitivity immune response in an individual.

Accordingly, the treatment of a hypersensitivity immune response of the present invention involves at least treatment of the individual in need thereof with an antigen unrelated to the "triggering" antigen and means to ensure co-availability/co-exposure/co-presence of the "triggering" antigen and the unrelated antigen to the target organ relevant for the natural exposure to the "triggering" antigen.

Thus, a main aspect of the invention relates to a method for treatment or prophylactic treatment of a hypersensitivity immune response in an individual in need thereof, such as to a method for bystander suppression of a hypersensitivity immune response in an individual in need thereof, wherein the immune response is triggered by an allergen upon the individual's exposure to a source material comprising said allergen and wherein;
i) the antigen is unrelated to the allergen(s) triggering the hypersensitivity immune response in the individual and is obtainable from or is derivable from said source material; and
ii) the antigen is administered in a therapeutically effective amount to said individual.

Alternatively worded, a main aspect relates to an antigen for use in treatment or prophylactic treatment of a hypersensitivity immune response in an individual in need thereof, such as to an antigen for use in bystander suppression of a hypersensitivity immune response in an individual in need thereof, wherein the immune response is triggered by an allergen upon the individual's exposure to a source material comprising said allergen and wherein
i) the antigen is unrelated to the allergen(s) triggering the hypersensitivity immune response in the individual and is obtainable from or is derivable from said source material; and
ii) the antigen is administered in a therapeutically effective amount to said individual.
   Still alternatively worded, a main aspect relates to the use of an antigen for the manufacturing of a medicament for use in treatment or prophylactic treatment of a hypersensitivity immune response in an individual in need thereof, such as to the use of an antigen for the manufacturing of a medicament for use in bystander suppression of a hypersensitivity immune response in an individual in need thereof, wherein the immune response is triggered by an allergen upon the individual's exposure to a source material comprising said allergen and wherein
iii) the antigen is unrelated to the allergen(s) triggering the hypersensitivity immune response in the individual and is obtainable from or is derivable from said source material; and
iv) the antigen is administered in a therapeutically effective amount to said individual.

In a subaspect thereof, it should be understood that wherein the method/antigen is for use in treatment of a hypersensitivity immune response in an individual, the individual has specific IgE antibodies, such as specific serum IgE antibodies, against the allergen(s). Moreover, it should be understood that this individual does not necessarily present clinical symptoms of the hypersensitivity immune response at the time of treatment, but the individual is sensitized to an allergen.

In another subaspect thereof, it should be understood that wherein the method/antigen is for use in prophylactic treatment of a hypersensitivity immune response in an individual, the individual does not have specific IgE antibodies, such as specific serum IgE antibodies, against the allergen at the time of initiating the prophylactic treatment. Therefore, in such aspects, the antigen shall be obtainable and/or derivable from source materials containing allergens to which the individual wants to be prevented from in the sense that sensitization and/or the eliciting of a hypersensitivity immune response is prevented or diminished in the individual upon the individual's exposure to the source material. Generally, it is observed that individuals with histories of atopic diseases among their relatives might be in risk of developing a hypersensitivity immune response and many of those individuals will be sensitized to the same allergens as noted in already sensitized individuals. As such, wherein the sub aspect relates to prophylactic treatment of a hypersensitivity immune response, the antigen is unrelated to an allergen of a pre-selected source material comprising an allergen to which a group of individuals have specific IgE antibodies against and is obtainable and/or is derivable from said pre-selected source material.

### Legends to Figures

Figure 1. An outline of the prophylactic SLIT treatment protocol with an unrelated antigen in mice.
Figure 2. Panel A represents result from proliferation of spleen cells isolated from Phl p versus buffer SLIT treated mice that have subsequently been i.p. immunized with OVA and Phl p together, whereas panel B shows the proliferation of spleen cells isolated from similar mice subsequently i.p. immunized with OVA, only. Each dot represents an individual mouse.
Figure 3. Panel A and B represent cytokine levels of IL-4 and IL-5, respectively, at day 5 as determined in the supernatant of the spleen cell culture derived from Phl p or buffer SLIT treated mice that have subsequently been i.p. immunized with OVA and Phl p together. Each dot represents an individual mouse.
Figure 4. Panel A represents result from proliferation of spleen cells isolated from Phl p versus buffer SLIT treated mice that have subsequently been i.p. immunized with Phl p and OVA together, whereas panel B shows the proliferation of spleen cells isolated from similar mice subsequently i.p. immunized with Phl p, only.
Figure 5. An outline of the prophylactic SLIT versus per-oral treatment with an unrelated antigen in mice is depicted.
Figure 6. The figure shows the proliferation of spleen cells from mice SLIT treated with Phl p extract either by the sublingual or the per-oral (intragastric gavache) route for two weeks. These treatments were followed by an intra-peritoneal injection with Phl p and OVA together. Following euthanization, the spleen cells were re-stimulated by a low dose of OVA (5µg/mL, panel A) or a high dose of OVA (125 µg/mL, panel B). Each dot represents an individual mouse.
Figure 7. An outline of the prophylactic SLIT treatment with an unrelated antigen in mice with clinically relevant readouts of a Th2-driven immune response is depicted.

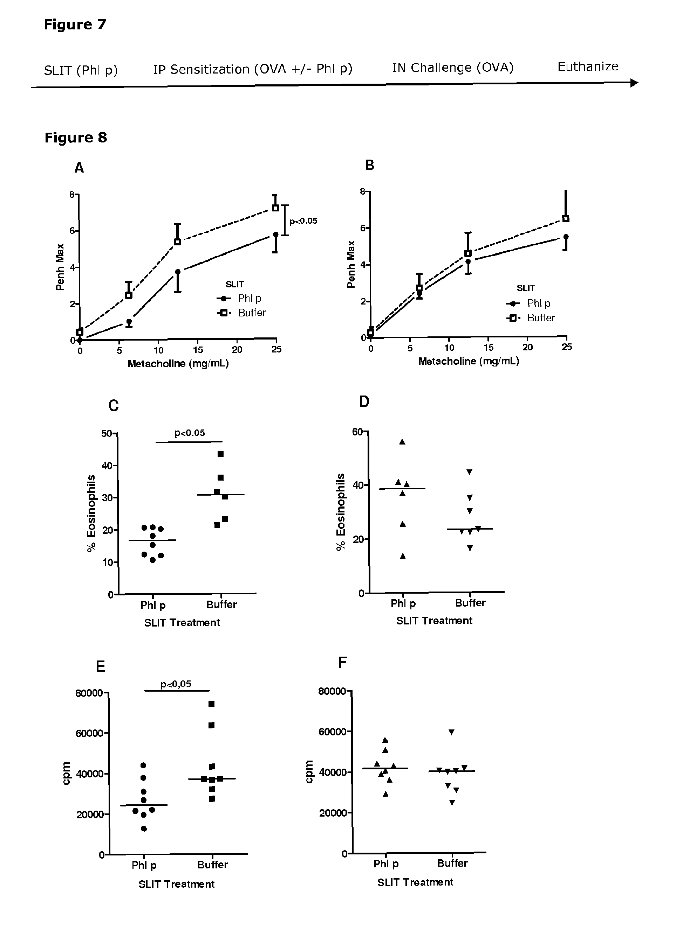

Figure 8. The figure shows the result from prophylactic SLIT treatment of naïve mice with an unrelated antigen prior to immunizing and intra-nasally challenging the mice to another antigen so as to induce clinically relevant readouts of allergic asthma. Panel A and B show the airway hyperresponsiveness in SLIT treated mice immunized with OVA and Phl p together and with OVA only, respectively. Panel C and D show the percentage of eosinophils in bronchoalveolar lavage fluid (BAL) obtained from SLIT treated mice immunized with OVA and Phl p together and with OVA only, respectively. Panels E and F show the in-vitro proliferation of cervical lymph node cells isolated from LIT treated mice immunized with OVA and Phl p together and with OVA only, respectively.
Figure 9. An outline of SLIT treatment with an unrelated antigen (phl p) in OVA antigen pre-sensitized mice developing an immune response against OVA antigen with clinically relevant readouts of a Th2-driven immune response.
Figure 10. Panel A and B show the fraction of eosinophils in the BAL fluid in OVA pre-sensitized mice SLIT treated with Phl p (unrelated antigen) versus mice SLIT treated with buffer, upon the subsequent intranasal challenge with OVA and Phl p together versus OVA alone, respectively.
Figure 11. Levels of IL-5 in BAL fluid obtained from OVA pre-sensitized mice and SLIT phl p treated mice versus SLIT buffer treated mice. Panel A refers to mice exposed to both OVA and Phl p (unrelated antigen) upon intranasal challenge. Panel B refers to mice exposed to OVA (antigen triggering an immune response) upon intranasal challenge.
Figure 12. Airway hyperresponsiveness in OVA pre-sensitized and SLIT phl p treated mice versus SLIT buffer treated mice. Panel A refers to mice exposed to both OVA and Phl p (unrelated antigen) upon intranasal challenge. Panel B refers to mice exposed to OVA (antigen triggering an immune response) upon intranasal challenge.
Figure 13. An outline of SLIT treatment with an unrelated antigen (OVA) in Phl p antigen pre-sensitized mice developing an immune response against Phl p antigens with clinically relevant readouts of a Th2-driven immune response.
Figure 14. Panel A and B show the number of sneezes in Phl p sensitized mice SLIT treated with OVA versus buffer SLIT treated mice upon intranasal challenge with Phl p. Panel A represents mice intra-nasally challenged to both Phl p and OVA and Panel B represents mice mice intra-nasally challenged to Phl p, only.
Figure 15. Panel A and B show the fraction of eosinophils in the BAL fluid in Phl p pre-sensitized mice SLIT treated with OVA (unrelated antigen) versus mice SLIT treated with buffer, upon the subsequent intranasal challenge with Phl p and OVA together versus Phl p alone, respectively.
Figure 16. Shows the levels of IL-5 in cell culture supernatants of cervical LN cells obtained from Phl p pre-sensitized mice and SLIT OVA treated mice versus SLIT buffer treated mice.
Figure 17. An outline of SLIT treatment with an unrelated antigen (phl p) in OVA antigen pre-sensitized mice developing an immune response against Phl p antigens with clinically relevant readouts of a Th2-driven immune response.
Figure 18. Panel A shows airway hyper responsiveness in OVA pre-sensitized and SLIT phl p treated mice (open circles) versus SLIT buffer treated mice (open triangles). Panel B shows the fraction of eosinophils in BAL fluid in OVA pre-sensitized and SLIT phl p treated mice (filled circles) versus SLIT buffer treated mice (filled triangles). Panel C shows in vitro proliferation of spleen cells from OVA pre-sensitized and SLIT phl p treated mice (filled circles) versus SLIT buffer treated mice (filled triangles)
Figure 19. Panel A shows the fraction of eosinophils in BAL fluid in mice that were SLIT treated with either Phl p 5 or buffer and subsequently i.p. sensitized with OVA and Phl p 5 together and then intranasally challenged with OVA. Panel B shows the in vitro proliferation of spleen cells from the same mice as above upon re-stimulation with OVA.
Figure 20. CIE diagram showing precipitates of serum IgE antibodies and antigens of a grass pollen extract of Pleum Pratense (radioactive + non-radioactive).
Figure 21. Allogram showing the number of individuals having various antigens (allergens) of a grass pollen extract of Pleum Pratense detected in their serum. Where, a precipate cannot be assigned to any of the scores; strong, moderate or weak, there is an empty space in the Allergogram. Empty spaces designate an unrelated antigen.
Figure 22 CIE diagram showing precipitates of serum IgE antibodies and antigens of a mite allergen extract of Dermatophagoides pteronyssinus (Der p) (radioactive + non-radioactive).

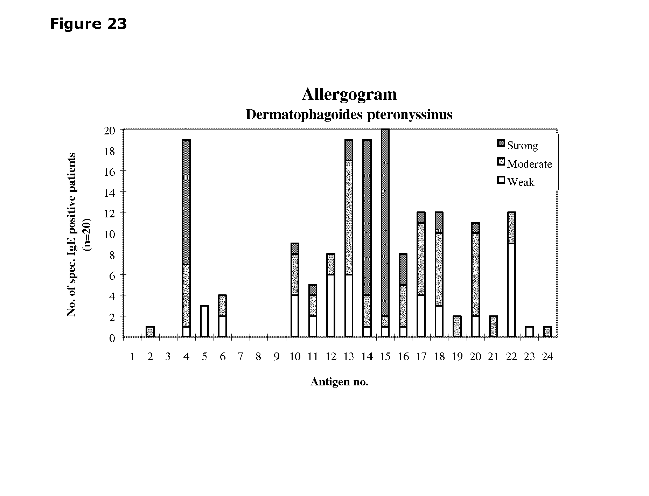

Figure 23. Allogram showing the number of individuals having various numbered antigens (allergens) of Dermatophagoides pteronyssinus (Der p) detected in their serum.
Figure 24. CIE diagram showing precipitates of serum IgE antibodies and antigens of a mite allergen extract of Dermatophagoides farinae (Der f) (radioactive + non-radioactive).

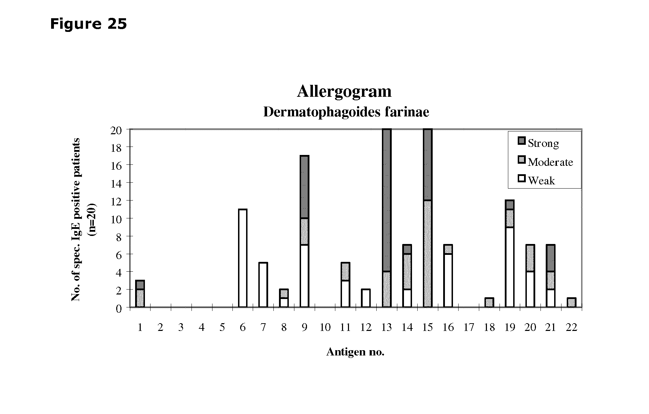

Figure 25. Allogram showing the number of individuals having various numbered antigens (allergens) of Dermatophagoides farinae (Der f) detected in their serum.

### Detailed Description

### Definitions

The following terms and phrases shall have the following meaning;

The term "a" or "an" refers to an indefinite number and shall not only be interpreted as "one" but also be interpreted in the meaning "some" or "several".

The phrase "an allergen" shall be interpreted as one or more allergen(s).

The term "allergen" is meant to designate an allergen capable of eliciting a hypersensitivity immune response in an individual, such as in an animal, such as in a human. The allergen may be a sensitizing allergen or a cross-reacting allergen.

The term "sensitizing allergen" is meant to designate an allergen capable of triggering the immune system to produce IgE antibodies in an individual.

The term "cross-reacting allergen" defines an allergen that responds to IgE antibodies originally created against a sensitizing allergen. That is to say that a cross-reacting allergen is an allergen capable of eliciting a hypersensitivity immune response due to cross-reactive IgE antibodies induced by a sensitizing allergen. For example the allergen Mal d 1 of apples is a protein homologous to Bet v 1 of birch tree pollen, and able to elicit a hypersensitivity immune response in an individual pre-sensitized to bet v 1 allergen due to its ability to trigger mast cells loaded with IgE anti-Bet v 1.

The term "major allergen" shall mean an allergen that causes sensitization in more than 50% of a population of patients having a hypersensitivity immune response triggered by exposure to an allergen source material comprising said allergen.

The term "minor allergen" shall mean an allergen that causes sensitization in less than 50% of a population of patients having a hypersensitivity immune response triggered by exposure to a allergen source material comprising said allergen.

The phrase "antigen unrelated to an allergen" and the term "(unrelated antigen" are interchangeable terms and are meant to designate a proteinaceous antigen distinct and different from an allergen, such as different from a sensitizing allergen as well as a cross-reacting allergen.

The phrase "antigen unrelated to an allergen triggering a hypersensitivity immune response in an individual" is meant to designate a proteinaceous antigen distinct and different from an allergen, such as different from a sensitizing allergen as well as a cross-reacting allergen capable of triggering a hypersensitivity immune response in the individual.

The phrase "antigen obtainable from a source material" is meant to designate that the antigen is isolated from the source material comprising the allergen or is otherwise isolated/obtained from another source material, but still identical to or at least essentially identical to the unrelated antigen present in the original source material comprising the allergen capable of triggering a hypersensitivity immune response in the individual.

The phrase "antigen derivable from a source material" is meant to designate an antigen identical to or at least essentially identical to the unrelated antigen present in the source material comprising the allergen capable of triggering a hypersensitivity immune response in an individual in need thereof, but reproduced by means of chemical and/or biological methods, such as by peptide synthesis and/or recombinant protein techniques according to methods well known in the art of proteins.

The term "antigen essentially identical to the unrelated antigen" is meant to designate that the antigen is slightly modified in relation to the unrelated molecule that is present or isolated from the source material comprising the allergen. Typical modifications are defined further herein.

The term "bystander suppression" is generally meant to encompass the ability to suppress an immune reaction in an individual towards one antigen (A) by treatment of the individual with another unrelated antigen (B).

The term "tolerance-induced bystander suppression" is generally meant to encompass the ability to suppress an immune reaction in an individual towards one antigen (A) by treatment of the individual with another unrelated antigen (B).

The term "co-availability" as used herein is meant to designate that the unrelated antigen is presented to the same target organ, such as to the same mucosa or skin, as reached by the "triggering" allergen through the natural exposure of a source material comprising the allergen. It does not necessarily mean that the unrelated antigen and the allergen are available or presented to the target organ at the exactly same time, but close enough in time to ensure that bystander suppression takes place. For example, the unrelated antigen is presented to/available at the target organ hours, days or weeks before, or after, the "triggering" allergen reaches the target organ. However, the unrelated antigen is preferably presented to the target organ within a period at least overlapping partly or entirely, such as coinciding partly or entirely, with the period where the triggering allergen is exposed to the same target organ.

The term "co-exposure" as used herein, is meant to encompass that the disease-eliciting antigen, such as the "triggering" allergen, and the unrelated antigen is exposed to the same target organ, e.g. the same mucosa upon the time where the individual is exposed to "triggering" allergen. It does not necessarily mean that the unrelated antigen and the sensitizing allergen are available or presented to the same body part at the same time, but close enough in time, to ensure that the bystander suppression takes place. For example, the unrelated antigen is presented to/available at the target organ hours, days or weeks before, or after, the "triggering" allergen reaches the target organ. However, the unrelated antigen is preferably presented to the target organ within a period at least overlapping partly or entirely, such as coinciding partly or entirely, with the period where the triggering allergen is exposed to the same target organ.

The term "immunotherapy" is meant to encompass therapy, wherein the therapeutically active agent (the immunomodulator) is an antigen. Immunotherapy usually encompass repeatedly administration of a sufficient dose of the antigen, usually in microgram quantities, over a prolonged period of time, usually for months or for years, wherein the antigen is administered daily, weekly, bi-weekly, or monthly.

The term "an individual" is meant to designate a mammal having an adaptive immune system, such as a human, a pet like a dog or a cat, and a farm animal like a horse or cattle.

The phrase "an individual in need of thereof" is meant to encompass an individual having a hypersensitivity immune response, an individual sensitized to an allergen as well as an individual in risk of being sensitized to an allergen or in risk of developing a hypersensitivity immune response. The individual may present clinically symptoms of a hypersensitivity immune response or the individual may only be sensitized to an allergen and not yet presenting clinically symptoms of a hypersensitivity immune response. An individual in risk of being sensitized to an allergen may be identified due to family histories of atopic individuals.

The phrase "an individual sensitized to an allergen" is meant to encompass an individual having specific IgE antibodies, such as serum antibodies, towards the allergen.

The phrase "prophylactic treatment" is meant to encompass treatment, such as by immunotherapy, of an individual with the aim to alleviate a hypersensitivity immune response or completely preventing the individual from developing a hypersensitivity immune response. Prophylactic treatment, such as prophylactic immunotherapy, is therefore initiated before the individual becomes sensitized to an allergen. This may be realized by initiating immunotherapy before the individual has raised detectable serum IgE antibodies capable of binding specifically to the sensitizing allergen or that any other biochemical marker indicative of a hypersensitivity immune response can be detected in biological samples isolated from the individual. Furthermore, prophylactic immunotherapy shall also designate immunotherapy initiated before the individual has evolved clinical symptoms of the disease, such as symptoms of allergic rhinitis or allergic asthma.

The term "treatment" refers to any type of treatment or prevention that imparts a benefit to a subject afflicted with or at risk of developing a hypersensitivity immune response to an allergen of interest, including improvement in the condition of the subject (e.g., in one or more symptoms), delay in the onset of symptoms or slowing the progression of symptoms, etc. As used herein, "treatment" is not necessarily meant to imply cure or complete abolition of symptoms, but refers to any type of treatment that imparts a benefit to a patient.

The phrase "therapeutically effective amount" is meant to designate an amount effective to treat, such as an amount sufficient to achieve the desirable effect. For example, a therapeutically effective amount is the total dose of an unrelated antigen administered during a period of immunotherapy in order to achieve the intended efficacy or the maximal dose tolerated within a give period. The total dosde may be divided into single doses administered daily, twice a week, weekly, every second or fourth week or monthly depending on the route of administration. The total dose may be administered in different concentrations. It is expected that a single dose is in the microgram range, such as in the range of 5 to 500 microgram dependent on the unrelated antigen.

The phrase "sensitized to an allergen" is generally meant to encompass that the individual has been exposed to an allergen in a manner that the individual's adaptive immune system displays memory to the allergen, such as has raised detectable IgE antibodies against the allergen or that t-cells stimulated in-vitro are able to proliferate under the presence of the sensitizing allergen.

The term "sublingual route" and the term "sublingual administration" are interchangeable terms meant to designate that the unrelated antigen is administered topically or trans-mucosally to the sublingual mucosa of an individual. Preferably, the unrelated antigen is provided as a pharmaceutical formulation suitable for this purpose, such as a tablet, e.g. fast-disintegrating tablet, or as a solution for drop-wise administration.

The term "adjuvant" refers to a substance that enhances the immune response to an antigen. Depending on the nature of the adjuvant it can promote either a cell-mediated immune response, humoral immune response or a mixture of the two.

### Summary on data provided herein

As shown in the Example section herein, the present inventors have studied bystander suppression by use of various experimental designs of murine models.

One of the experimental designs is a prophylactic murine model and is used in Examples 1 and 2. This model allows for investigating if prophylactic treatment with one antigen is able to downregulate a systemic inflammatory immune response caused by anotner antigen (triggering antigen) to which the first antigen is unrelated to. Moreover, this model allows for investigating if the downregulation is dependent on the presence of the unrelated antigen at the systemic challenge.

As a first step, naïve mice receive sublingual immunotherapy (SLIT) with the unrelated antigen in order to induce tolerance. Subsequently, the effect of the SLIT treatment is evaluated by inducing a systemic immune response by injecting the "triggering" antigen (i.e. allergen) adsorbed to alum intraperitoneally (i.p.). The immune response thus generated is generally characterized by spleenic T-cell proliferation and production of Th2 cytokines upon *in vitro* re-stimulation with the allergen. Therefore, the immune response generated in this simple prophylactic murine model reflects the human situation, where the individual undergoes prophylactic immunotherapy with the aim of preventing sensitization to a Th2-inducing allergen and/or preventing an immune response triggered by a Th2-inducing allergen.

To study bystander suppression, one group of mice is challenged with both the allergen and the unrelated antigen at the i.p. injection. Bystander suppression will be obtained in the mice, if splenic T-cells derived from mice treated sublingually with the unrelated antigen show reduced T-cell proliferation upon in vitro re-stimulation with the Th2-inducing allergen compared to T-cells derived from mice treated sublingually with placebo (buffer). In the current examples, two single antigens, OVA and Phleum p 5 allergen of grass pollen as well as a mixture of Pheum p allergens (aqueous allergen extract of grass pollen of the species Phleum Pratense) have been used as exemplary unrelated antigens. Two different Th2-inducing allergens, OVA and Phl p extract, have been used as exemplary allergens in triggering a Th2-like immune response in the murine models. For example, where OVA is applied as the unrelated antigen, the mice are challenged with the Phl p allergen extract, and vice versa with OVA as the challenge allergen.

Another experimental design of the prophylactive murine model is adopted in example 3 and 6 with the aim of demonstrating bystander suppression of airway inflammation and thus demonstrating bystander suppression of a clinically relevant immune response, as clinically relevant parameters like the fraction of eosinophils in the lung and airway hyper-reactivity are assessed. In this model, the i.p. injection that follows after the prophylactic SLIT treatment can be regarded as a sensitizing step, because it is followed by an intranasal challenge with the same allergen used at the i.p. sensitizing step. The additional step of intranasal challenge solely serves to induce airway inflammation clinically relevant to allergic asthma and rhinitis.

To study bystander suppression, one group of mice is challenged to both the allergen and the unrelated antigen at the i.p. injection. Bystander suppression will be obtained in mice sublingually treated with the unrelated antigen, if the mice show reduced eosinophilia and/or reduced airway hyper-reactivity compared to mice treated sublingually with placebo (buffer).

An experimental design of a therapeutic model mice model is used in Examples 4 and 5. The mice are first sensitized to the allergen by i.p. injection and subsequently treated by sublingual immunotherapy with an unrelated antigen. To study bystander suppression of a clinically relevant immune response, the mice are then challenged by intranasal installation of the allergen, either alone or together with the unrelated antigen with the aim of developing airway inflammation as may be observed by increased fraction of eosinophils in the lung, increased airway hyper-reactivity or increased sneezing, all relevant clinical symptoms of allergic asthma and rhinitis. Bystander suppression will be obtained in mice sublingually treated with the unrelated antigen, if the mice show reduced eosinophilia and/or reduced airway hyper-reactivity or sneezing compared to mice treated sublingually with placebo (buffer).

The murine models used herein are well known methods used in the field of studying specific allergen immunotherapy (J Brimnes et al, Clinical and Experimental Allergy 37, 488-497, 2007) and are models reasonably predictive for the efficacy in treatment of humans having the same immunological and clinically readouts as presented in the murine models used herein. Notably, the murine model presents many of the clinical and immunological features associated with human allergic rhinitis and asthma, including sneezing, increased airway hyperreactivity, influx of eosinophils as well as elevated levels of IL-5 and antigen specific IgE both locally and systemically (p 489 of J Brimnes et al 2007). The validity of the murine model of Brimnes et al 2007 has been confirmed in the Editorial of the publication in which the Brimnes article appeared, cf. "New allergy intervention strategies: hitting the mucosal road", U Wiederrnann et aI., Clinical and Experimental Allergy 37, 473-475, 2007.

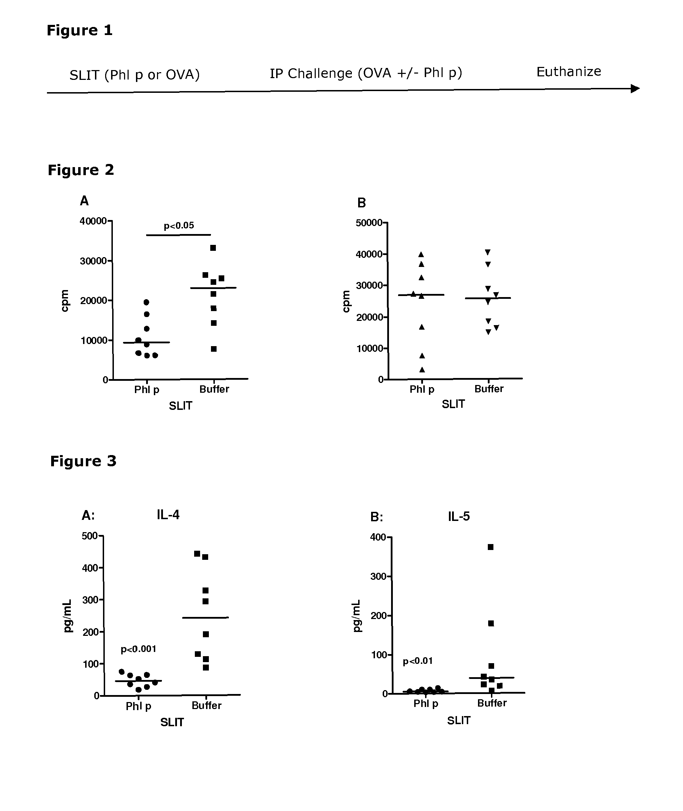

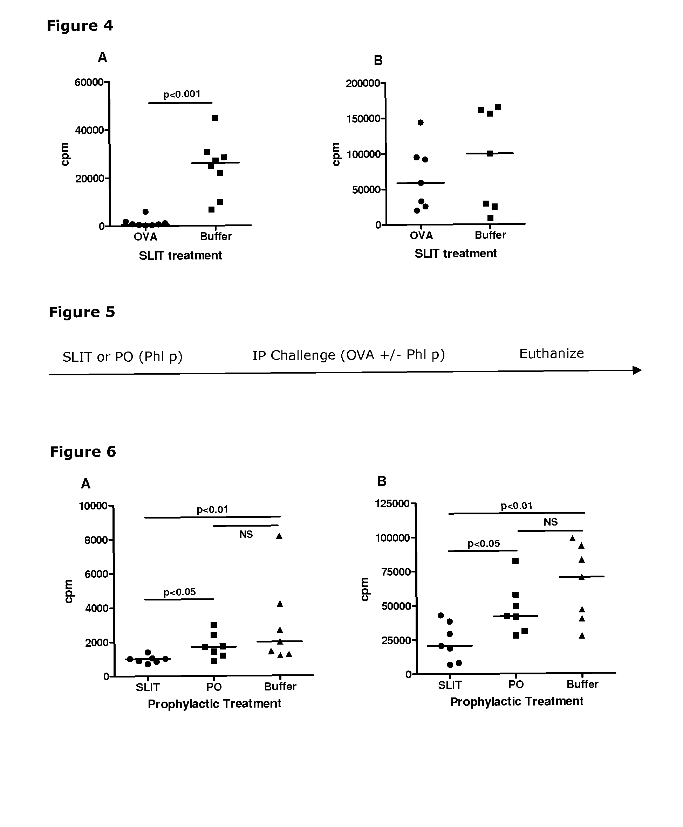

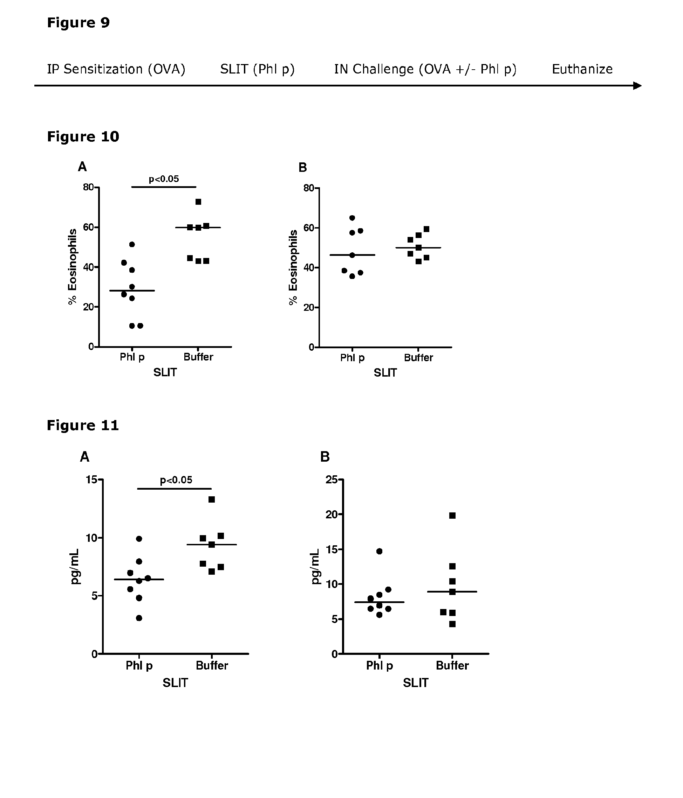

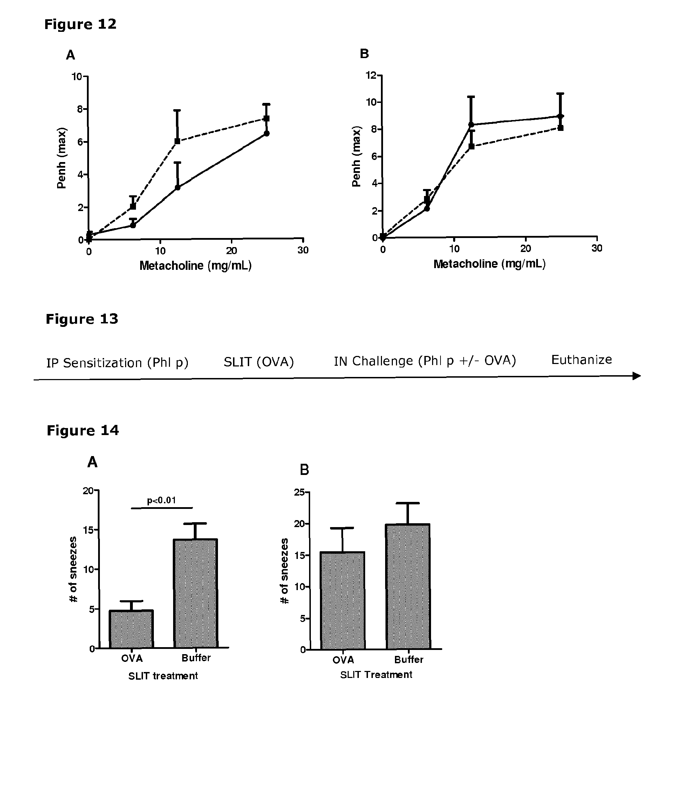

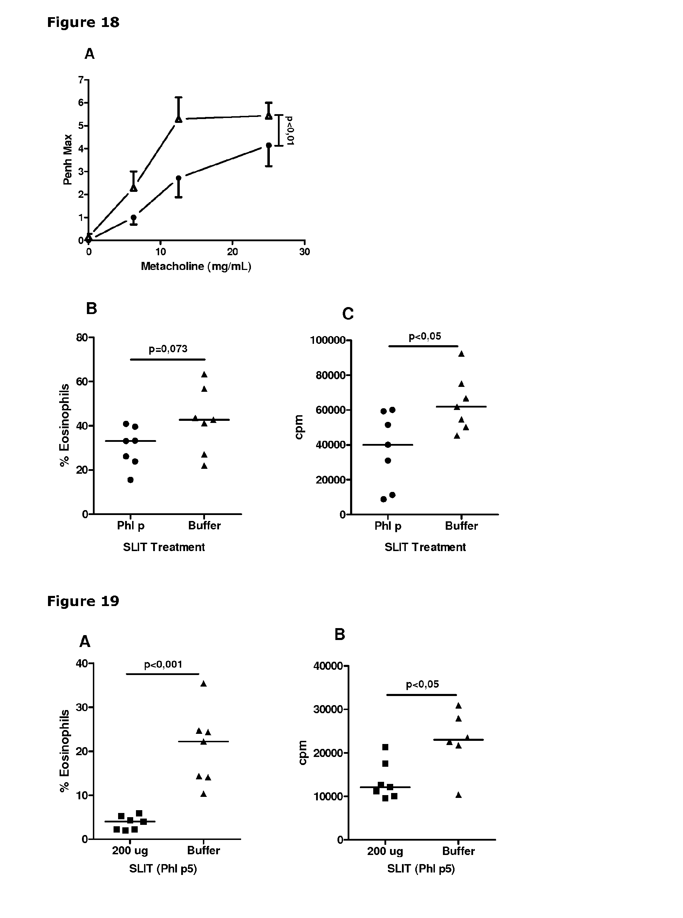

The results presented herein may be summarized as follows: Figures 2A and 3A shows bystander suppression of T-cell proliferation and of the Th2 cytokine Il-4 generation, respectively, in the prophylactic murine model using OVA as the "triggering allergen" and Phl p as the unrelated antigen. Figure 4A shows bystander suppression of T-cell proliferation in the prophylactic murine model using Phl p extract as the "triggering allergen" and OVA as the unrelated antigen. Figures 5A and B both show improved bystander suppression of T-cell proliferation rate in mice sublingually treated with unrelated antigen versus mice per-orally treated with unrelated antigen. The difference between A and B is the dose of OVA used in restimulating the spleen T-cells. Figures 8A, C and E show bystander suppression of airway hyperreactivity, influx of eosinophils and T-cell proliferation rate, respectively in the prophylactic murine model using OVA as the "triggering allergen" and Phl p extract as the unrelated antigen. Figures 10A, 11A and 12A show bystander suppression of the influx of eosinophils, IL-5 cytokine levels and airway hyper responsiveness in the therapeutic murine model using OVA as the "triggering" allergen and Phlp extract as the unrelated antigen. Figures 14A, 15A and 16 show bystander suppression with respect to the number of sneezes, influx of eosinophils and levels of IL-5 cytokine, respectively, in the therapeutic murine model using "Phl p extract" as the "triggering" allergen and OVA as the unrelated antigen. Figure 18A, B and C show bystander suppression of airway hyper responsesiveness, influx of eosinophils in BAL and T-cell proliferation rate, respectively, in the prophylactic murine model using OVA as the "triggering allergen" and Phl p extract as the unrelated antigen. Figure 19A and B show bystander suppression of influx of eosinophils in BAL and T-cell proliferation rate, respectively, in the prophylactic murine model using OVA as the "triggering allergen" and Phl p 5 (single antigen) as the unrelated antigen.

As may be noted, bystander suppression could only be demonstrated where the mice also are challenged to the unrelated antigen when challenged to the "triggering allergen". Therefore, figures 2B, 3B, 8B, 8D, 8F, 10B, 11B, 12B, 14B and 15B does not show bystander suppression as these figures refers to mice only challenged to the "triggering" allergen. Also it might be noted that an antigen usually defined as an allergen (Phl p 5 or Phl p extract) is presented herein as exemplary unrelated antigens. However, such allergens are still exemplary unrelated antigens in the sense that the mice are not sensitized to those allergens, but sensitized to OVA in the experiments wherein Phl p 5 or Phl p extract are used as the unrelated antigen.

Thus, the present inventors have provided numerous data pointing to the fact that an antigen unrelated to the "triggering" allergen is able to provide bystander suppression of a hypersensitivity immune response, such as airway inflammation. Furthermore, the numerous data provided herein indicates that bystander suppression also is achievable by use of other not yet tested unrelated antigens and that bystander suppression also can be demonstrated with respect to hypersensitivity immune response of the gastro-intestinal tract (food allergy) or of the skin (atopic dermatitis or contact dermatitis). Bystander suppression of a hypersensitivity immune response triggered by a dietary allergen, like an allergen of peanut, milk, egg or soyabean, may be studied using the murine model outlined in Example 8 and bystander suppression of a hypersensitivity immune response triggered by an allergen associated with contact dermatitis or atopic dermatitis may be studied using the murine model outlined in Example 9.

### Unrelated antigen and source materials

As mentioned, uses and methods of the present invention encompasses the administration of an antigen unrelated to the allergen (i.e. the one or more allergen(s)) triggering a hypersensitivity response in an individual and wherein the same antigen is obtainable from or is derivable from a source material comprising the allergen(s).

In principle any antigen present in the source material can candidate as an unrelated antigen of the present invention as long as the unrelated antigen is different from and distinct to the one or more allergen(s) triggering a hypersensitivity immune response in the particular individual in need of treatment thereof. Therefore, an unrelated antigen of the present invention might be identified by determining whether the unrelated antigen can or cannot, at least in detectable levels, bind to IgE antibodies, such as serum IgE antibodies, obtained from the individual to be treated. Alternatively, and in particularly in embodiments related to prophylactic treatment, it might be determined whether an unrelated antigen of the source material can or cannot, at least in detectable levels, bind to IgE antibodies, such as serum IgE antibodies, obtained from a group of individuals that has specific IgE antibodies, such as specific serum IgE antibodies against an allergen of a pre-selected source material of interest, e.g. grass pollen.

Thus, in some embodiments of the invention, the antigen is unrelated to the "triggering" allergen(s) of the source material in the sense that the antigen cannot, at least in detectable levels, bind to IgE antibodies, such as serum IgE antibodies, obtained from the individual in need of being treated. In other embodiments of the invention, the antigen is unrelated to the "triggering" allergen(s) in the sense that the unrelated antigen cannot, at least in detectable levels, bind to IgE antibodies, such as serum IgE antibodies, obtained from a group of individuals having specific antibodies against the allergen(s). This is also true where the same unrelated antigen might be used for treatment of several individuals in need thereof.

Therefore, in preferred embodiments of the invention, the antigen is unrelated to the "triggering" allergen(s) in the sense that the antigen cannot, at least in detectable levels, bind to IgE antibodies, such as serum antibodies, obtained from a group of individuals having specific IgE antibodies, such as serum IgE antibodies against said allergen(s).

Specific IgE antibodies to an allergen may be tested by well known methods in the art, such as by use of the RAST test that is a radioimmunoassay test to detect specific IgE antibodies to suspected or known allergens. In such test the suspected allergen is bound to an insoluble material and the patient's serum is added. If the serum contains antibodies to the allergen, those antibodies will bind to the allergen. Radiolabeled anti-human IgE antibody is added where it binds to those IgE antibodies already bound to the insoluble material. The unbound anti-human IgE antibodies are washed away. The amount of radioactivity is proportional to the serum IgE for the allergen.In recent years a more superior test named the ImmunoCAP Specific IgE blood test, which in the literature may also be describe as: CAP RAST, CAP FEIA (fluorenzymeimmunoassay), and Pharmacia CAP. The quantitatively detection limit of such test may be as low as about 0.1kU/l. For use in the context of the present invention, the quantitative detection limit may be below 1kU/l, such as below 0.5 kU/l, such as below 0.3kU/l where the ImmunoCAP® Specific IgE blood test or a comparable test is used.

Preferably, the antigen is unrelated to said allergen(s) in the sense that the individual in need of treatment and/or the group of individuals is/are tested negative for specific IgE antibodies, such as serum IgE antibodies, capable of binding to the antigen. That is to say that the IgE antibodies are at least not detectable or at least not present in quantifiable levels.

Obviously, the antigen might also differentiate from the allergen(s) in the sense that the antigen cannot induce a hypersensitivity immune reaction in the individual in need of treatment and/or the group of individuals. As such, in some embodiments of the invention, the antigen is unrelated to the allergen(s) in the sense that the antigen cannot initiate an immediate skin reaction in the individual in need of treatment and/or the group of individuals upon conducting skin prick testing with various concentrations of the antigen.

Also in still other further embodiments of the invention, the antigen is unrelated to the allergen(s) in the sense that the antigen cannot induce histamine release in an in-vitro basophil/mast cell assay using blood from the individual to be treated or in from a group of individuals.

As mentioned, in some embodiments of the invention, the antigen is unrelated to an allergen defined by the IgE antibodies obtained from a group of individuals rather than from the individual to be treated. A representative amount of such a group may vary. It is though anticipated that a group of individuals consist of a number of individuals of at least 4 individuals, such as of at least 5, 10, 15, 20, 30, 40, 50 or at least 100 individuals. Where it is desirable to select an antigen for use in treatment of a greater group of individuals, the antigen might be different from all sensitizing allergens or cross-reacting allergens thereof to which a group of patients have specific IgE antibodies raised against. Here it might be relevant to test a group of patients of at least 30 patients, more preferably 40, even more preferably 50 individuals. Thus, in interesting embodiments of the invention, a group of individuals have a number of individuals in the range of 30 to 1000 individuals, such as of 30 to 500, such as of 30 to 400, such as of 30 to 300, such as of 30 to 250, such as of 30 to 100.

Also, an unrelated antigen may be identified by determining whether the antigen has a distinct antigenic determinant in relation to the allergen, such as sharing B-cell and/or T-cell epitopes, including conformational epitopes and linear epitopes, with the allergen in question. Therefore, in preferable embodiments of the invention, the antigen is unrelated to said allergen(s) in the sense that the antigen does not share any B-cell or T-cell epitopes with said allergen(s). Epitopes can be "mapped," the process of identifying and characterizing the minimum molecular structures that are able to be recognized by the immune system, according to methods known in the art, e.g. protein microarrays, ELISPOT or ELISA techniques, etc. In some embodiments, T cell epitopes which bind to MHC class II molecules are mapped with the use of Tetramer Guided Epitope Mapping (TGEM). See U.S. Patent No. 7,094,555 to Kwok et al., which is incorporated by reference herein in its entirety. Epitopes may also be predicted based upon computer modeling, e.g., the TEPITOPE program. See, e.g., Kwok et al., Trends in Immunology, 2001, 22(11): 583-588.

Therefore, in preferable embodiments of the invention, the unrelated antigen has an amino acid sequence having less than 70% identity, preferable less than 65, 60, such as less than 65, less than 60%, less than 55%, such as less than 50%, such as less than 45%, such as less than 40% identity, such as less than 30%, such as less than 25%, such as less than 20% identity to the amino acid sequence of the allergen.

Furthermore, an unrelated antigen for use in the present invention may preferable not, such as must not bind to IgE antibodies, such as serum IgE antibodies, obtained from the individual to be treated.

Also the unrelated antigen does not bind, at least not in detectable levels, to IgG antibodies, e.g. rabbit anti IgG, raised after immunization of an animal with one or more or all allergen(s) of a pre-selected source material.

According to the invention, the unrelated antigen is an antigen found in a source material comprising the one or more allergen(s) capable of triggering a hypersensitivity immune response in an individual in need of treatment or in a group of individuals having specific IgE antibodies, such as serum IgE antibodies, to one or more allergens of a pre-selected source material. However, it is envisaged that the unrelated antigen need not be completely identical to the antigen found in the allergen source material. Therefore, the unrelated antigen of the invention might be more or less essentially identical, such as particularly essentially identical, to the original unrelated antigen selected/detected in the source material. For example, the unrelated antigen might be a homologous antigen of the originally detected/selected unrelated antigen. Therefore, in interesting embodiments of the invention, the unrelated antigen is an antigen present in the source material in question or a homologous antigen thereof, such as a variant thereof. That is to say that the antigen is a variant of the originally antigen obtainable or derivable from the source material.

Preferably, the source material is a naturally occurring source material like an environmental source material (e.g. pollen, animal dander, venoms and latex) or a dietary source material (e.g. nuts and cereals). It should be understood that an unrelated antigen of the present invention is obtainable from the source material in the sense that the unrelated antigen or a homologous antigen thereof is isolated from the source material comprising the "triggering" allergen or alternatively isolated from another source material comprising the same unrelated antigen or a homologous antigen thereof. Thus, in some embodiments, the antigen is obtainable from or is derivable from more than one source material each containing the same unrelated antigen. Still, alternatively, the unrelated antigen as present/found in a source material is reproduced by means of chemical and/or biological methods, such as by peptide synthesis and/or recombinant protein techniques according to methods well known in the art of proteins or other methods suitable for generating proteinaceous antigens.

An unrelated antigen found in a source material may be identified by use of well known protein separation techniques bearing in mind that proteins are easily denatured and the three-dimensional structure is easily lost. First of all, the source material comprising the allergen shall be identified. The source material may then be subjected to various extraction protocols, preferably where focus is on isolating a water-soluble unrelated antigen. For example, the unrelated antigen may be extracted from the source material comprising mainly aqueous extraction solvents. That is to say that the unrelated antigen is present in an aqueous extraction fraction of the source material, such as being eluted in an aqueous extraction solvent during chromatographic extraction and purification. Lipid extraction solvents may still be used for the purpose of defatting the source material or removing undesirable matter. Various fractions from the extraction procedure may then be subjected to methods suitable for detecting the unrelated antigen. Crossed radio-immuno-electrophoresis (CRIE) is a suitable method for this purpose. In brief, a suitable method for providing an unrelated antigen is to provide water-soluble protein extracts of a source material, e.g. pollen, which are then separated by electrophoresis, followed by another electrophoresis through a gel containing IgG antibodies raised against the water-soluble protein extract in question. IgG antibodies can be raised by immunising rabbits. The resulting antibody: antigen complexes (Ab:Ag complexes) can then be visualized by Coomasie blue staining or be incubated with sera from pollen sensitized individuals followed by anti human IgE antibodies, in order to visualize the allergens of the pollen extract. The antigens that are not bound by IgE antibodies from patient's sera are considered to be candidates to an unrelated antigen.

Example 7 herein relates to the detection of unrelated antigens in three difference source materials, namely in grass pollen and in two different species of house dust mites. As noted, several candidates can be detected by means of the procedure outline above.

It should also be understood that where the same unrelated antigen is found in different source materials, the unrelated antigen can be used in the treatment of hypersensitivity immune reactions triggered by exposure of allergens present in more than one source material. Therefore, advantageously, an unrelated antigen is selected among unrelated antigens commonly found in various source materials. For example, where the same unrelated antigen is found in pollen of different genus, such as grass pollen of the genus Phleum and Lolium, the unrelated antigen is advantageously selected among the common unrelated antigens detected in grass pollen of the genus Phleum and Lolium. Furthermore, where the unrelated antigen including a variant, such as a homologous antigen thereof, is present in a grass pollen species as well as in a birch pollen species, the unrelated antigen is advantageously selected among the common unrelated antigens present in both the grass pollen species and the birch pollen species.

Accordingly, in still further interesting embodiments of the invention, the unrelated antigen is obtainable from or is derivable from more than one source material each containing the same unrelated antigen or essentially the same unrelated antigen.

For example, the unrelated antigen might be isolated or derived from grass pollen, but used in the treatment of a hypersensitivity immune reaction triggered by one or more allergens of birch pollen.

As mentioned, the unrelated antigen might be essentially identical to the originally one detected in the allergen source material, e.g. the unrelated antigen is slightly modified in relation to the originally found unrelated antigen. For example, the originally found antigen is slightly modified before, during or subsequent to the isolation procedure of the originally detected unrelated antigen. More specifically, the originally detected antigen is slightly modified during the chemical and/or biological reproduction. The wording "slightly modified" in the present context is meant to designate that the modified antigen is essentially the same as the originally detected unrelated antigen. At least, it should be understood that the unrelated antigen might be modified to an extent where bystander suppression of a hypersensitivity immune response is still achieved.

Therefore, the unrelated antigen of the present invention is said to be derivable from the source material comprising the "triggering" allergen in the sense that the unrelated antigen is essentially identical to the unrelated antigen found in the source material, but slightly modified. That is to say that the unrelated antigen is an antigen variant of the originally unrelated antigen, such as a homologous antigen of the originally unrelated antigen.

For example, the originally detected antigen is chemical modified or biological modified so as to modify its solubility properties, bioavailability properties or stability. Examples are antigen variants with chemical modifications, such as N- or O-linked glycosylation or derivatizing of the N-terminus or of thiol groups of the unrelated antigen. Other examples are antigen variants that are biologically modified, such as by post-translational modifications, such as for example; glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

It is envisaged that the antigen variant of an originally unrelated antigen has an amino acid chain length having a number of amino acids in the range of 50% to 150%, such as in the range of 70% to 130% in relation to the originally found unrelated antigen, such as preferable in the range of 75% to 125%, such as even more preferable in the range of 80% to 120%, such as still more preferable in the range of 85% to 115% in relation to the originally found unrelated antigen. Still more preferable, the antigen variant has an amino acid chain length having a number of amino acids in the range of 90% to 110%, such as 95% to 105%, such as 98% to 102% in relation to the originally found unrelated antigen.

The term "homologue antigen" or an "antigen variant" is meant to include an antigen that has significant homology or identity to the original antigen. Significant homology or identity means at least 50 percent, 55 percent, 60 percent, 65 percent, 70 percent, 75 percent, 80 percent, 85 percent, 90 percent, 95 percent, 98 percent and/or 100 percent homology or identity with the amino acid sequence of the original antigen. Such homologues can also be identified by having significant identity at the nucleotide sequence level (i.e., at least 50 percent, 55 percent, 60 percent, 65 percent, 70 percent, 75 percent, 80 percent, 85 percent, 90 percent, 95 percent, 98 percent and/or 100 percent or identity with another nucleotide sequence).

Exemplary protein variants of an originally found/isolated unrelated antigen may be a substitution variant, deletion variant, duplication variant, insertion variant, insertion-deletion variant or frame shift variant or any other suitable variant.

Bearing in mind that an antigen variant, such as a homologous antigen of the unrelated antigen, may be essentially identical to the originally detected/isolated unrelated antigen, it is anticipated that in some embodiments only a limited number of the amino acids in the amino acid sequence of the originally found/isolated unrelated antigen is deleted, substituted, added or derivatized. For example, it might be anticipated that less than 20, such as less than 15, 10, 8, 6, 5, 4, or 3 amino acids of the amino acid sequence of the originally detected/isolated unrelated antigen be substituted by another amino acids. Furthermore, less than 20, such as less than 15, 10, 8, 6, 5, 4, or 3 amino acids be added to or deleted from the amino acid sequence of the originally detected/isolated unrelated antigen. Also it is anticipated that less than 15, 10, 8, 6, 5, 4, or 3 amino acids of the originally detected/isolated unrelated antigen be subjected to derivatization, such as glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

More particularly, in some embodiments of the invention, the antigen variant, such as a homologous variant, has no more than 30% of the amino acids of the originally detected unrelated antigen subjected to substitution, deletion, duplication, insertion, or a mixture thereof. More preferably no more than 25%, such as even more preferably no more than 20%, such as no more than 15%, such as no more than 10%, such as no more than 5% of the amino acids of the originally detected unrelated antigen are subjected to substitution, deletion, duplication, insertion, or a mixture thereof.

Also it is anticipated that less than 20%, such as less than 15%, less than 10, 8, 6, 5, 4, or 3% amino acids of the originally detected/isolated unrelated antigen be subjected to derivatization, such as glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

Furthermore, a homologous antigen or an antigen variant may in some embodiments refers to an antigen having high amino acid sequence similarity to the originally detected/isolated unrelated antigen, such as having similar protein folding. For example, the percentage of amino acid sequence identity is high and may be determined by comparing two aligned amino acid sequences over a comparison window, preferably comparing full-length amino acid sequences, where the variant protein of the unrelated antigen may comprise additions or deletions (e.g., gaps or overhangs) as compared to the reference sequence (the originally detected/isolated unrelated antigen (which does not comprise additions or deletions) for optimal alignment of the two sequences. The percentage identity is calculated by determining the number of positions at which the identical amino acid residue occurs in both sequences to yield the number of matched positions, dividing the number of matched positions by the total number of amino acids in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, Wis.), or by inspection. Given that two sequences have been identified for comparison, GAP and BESTFIT are preferably employed to determine their optimal alignment. Typically, the default values of 5.00 for gap weight and 0.30 for gap weight length are used.

The term "high amino acid sequence identity" between variant antigen (homologous antigen) and originally detected/isolated antigen refers to a variant antigen having an amino acid sequence having at least 70 percent, such as at least 75 percent, such as at least 80 percent sequence identity or homology, preferably at least 85 percent, more preferably at least 90 percent and most preferably at least 95 percent, even more preferably, at least 96 percent, 97 percent, 98 percent or 99 percent sequence identity or homology compared to the originally detected/isolated antigen.

Alternatively expressed, the unrelated antigen is a protein variant, such as a homologous antigen having an amino acid sequence homology of least 70 percent, such as at least 75 percent, such as at least 80 percent sequence identity or hopmology, preferably at least 85 percent, more preferably at least 90 percent and most preferably at least 95 percent, even more preferably, at least 96 percent, 97 percent, 98 percent or 99 percent sequence identity compared to the originally detected/isolated antigen.

In currently interesting embodiments of the invention, the unrelated antigen is identical to its naturally occurring form including salts thereof and derivates thereof. In still currently interesting embodiments of the invention, the unrelated antigen is a homologous antigen thereof having an amino acid chain length in the range of 50% to 150%, such as in the range of 70% to 130% and at least 70 percent sequence identity or homology, such as at least 80 percent, such as at least 85 percent, such as at least 90 percent sequence identity in relation to the originally found unrelated antigen (as isolated or as detected).

In more interesting embodiments of the invention, the unrelated antigen is a variant antigen, such as a homologous antigen thereof, having an amino acid chain length in the range of 80% to 120% and at least 70 percent sequence identity or homology, such as at least 80 percent, such as at least 85 percent, such as at least 90 percent sequence identity in relation to the originally found unrelated antigen (as isolated or as detected).

In still more interesting embodiments of the invention the unrelated antigen is a variant antigen, such as a homologous antigen thereof, having an amino acid chain length in the range of 90% to 110% and at least 70 percent sequence identity, such as at least 80 percent, such as at least 85 percent, such as at least 90 percent sequence identity or homology in relation to the originally found unrelated antigen (as isolated or as detected).

In still more interesting embodiments of the invention, the unrelated antigen is a variant antigen, such as a homologous antigen thereof, having an amino acid chain length in the range of 95% to 105% and at least 70 percent sequence identity or homology, such as at least 80 percent, such as at least 85 percent, such as at least 90 percent, such as at least 95 percent sequence identity or homology in relation to the originally found unrelated antigen (as isolated or as detected).

It should be understood that in all of the interesting embodiments of the invention, the antigen variant, such as a homologous variant, has no more than 30% of the amino acids of the originally detected unrelated antigen subjected to substitution, deletion, duplication, insertion, or a mixture thereof. More preferably no more than 25%, such as even more preferably no more than 20%, such as no more than 15%, such as no more than 10%, such as no more than 5% of the amino acids of the originally detected unrelated antigen are subjected to substitution, deletion, duplication, insertion, or a mixture thereof.

As a proteinaceous antigen, the unrelated antigen inherently possesses immunogenic properties and is recognized by the individual's immune system as foreign. Thus, an unrelated antigen is capable of inducing specific IgG antibodies, such as IgG₄ or IgG₁ antibodies, IgM or IgA antibodies capable of binding specifically to the unrelated antigen depending on the route of exposure/administration.

The unrelated antigen is a protein by nature, including a glycoprotein, a lipoprotein and post-translational derivatives thereof e.g. phospho-proteins. The unrelated antigen may also be provided as an immunogenic peptide, including a glycopeptide, a lipopeptide and post-translational derivatives thereof e.g. phospho-peptides, provided that this peptide is not identical or homologous to a peptide fragment of an allergen.

Also, in more preferred embodiments of the invention, the unrelated antigen is a water-soluble antigen. In still more interestingly embodiments of the invention, the unrelated antigen is an antigen being co-eluted/co-extracted with the allergen(s) of the source material upon exposing the source material to biological fluids, like fluids lining a mucosa of the respiratory tract or gastro-intestinal tract, such as nasal secretes, saliva, lung secretes or intestinal fluids. Also the unrelated antigen is preferable an antigen being co-eluted/co-extracted with the allergen(s) of the source material upon suspending/dissolving the source material in an aqueous solution having a composition reflecting a biological fluid, such as an aqueous solution having pH in the range of 6 to 8, such as particularly in the range of 6.5 to 7.5, optionally with the presence of saline and/or presence of relevant enzymes. For example, in interesting embodiments of the present invention, the antigen is selected among antigens able to be co-extracted with the allergen(s) upon subjecting the source material to an aqueous saline solution having pH in the range of 6 to 8, such as preferably in the range of 6.5 to 7.5 for a period not exceeding 60 minutes, such as preferably not exceeding 45, 30, 20, 15, 10 or 6 minutes.

According to the invention, the antigen of the invention is unrelated to the allergen triggering the hypersensitivity immune response. Generally, not all of the allergens of a source material are able to trigger a hypersensitivity immune response. Usually, only major allergens are involved. That is to say that in more interesting embodiments of the invention, the antigen of the invention is unrelated to major allergens present in a source material. However, in other interesting embodiments of the invention, the antigen is unrelated to all allergens of a source material irrespective of those being a major or a minor allergen. That is to say that the unrelated antigen is not an allergen, such as an allergen present in an allergen-containing environmental source material or an allergen-containing dietary source material.

It might be understood that the specific source material is connected to the specific allergen triggering the hypersensitivity immune response. Thus, current interesting embodiments of the invention relate to;
I) A method for treatment or prophylactic treatment of a hypersensitivity immune response of the respiratory tract in an individual in need thereof, such as to a method for bystander suppression of a hypersensitivity immune response of the respiratory tract in an individual in need thereof, wherein the immune response is triggered by an allergen upon the individual's exposure to an airborne environmental source material comprising said allergen and wherein; i) the antigen is unrelated to the allergen(s) triggering the hypersensitivity immune response in the individual and is obtainable from or is derivable from said source material; and ii) the antigen is administered in a therapeutically effective amount to said individual.
   Alternatively worded, the embodiment relates to an antigen for use in treatment or prophylactic treatment of a hypersensitivity immune response of the respiratory tract in an individual in need thereof, such as to an antigen for use in bystander suppression of a hypersensitivity immune response of the respiratory tract in an individual in need thereof, wherein the immune response is triggered by an allergen upon the individual's exposure to an airborne environmental source material comprising said allergen and wherein i) the antigen is unrelated to the allergen(s) triggering the hypersensitivity immune response in the individual and is obtainable from or is derivable from said source material; and ii) the antigen is administered in a therapeutically effective amount to said individual.
II) A method for treatment or prophylactic treatment of a hypersensitivity immune response of the gastro-intestinal tract in an individual in need thereof, such as to a method for bystander suppression of a hypersensitivity immune response of the gastro-intestinal tract in an individual in need thereof, wherein the immune response is triggered by an allergen upon the individual's exposure to a dietary source material comprising said allergen and wherein; i) the antigen is unrelated to the allergen(s) triggering the hypersensitivity immune response in the individual and is obtainable from or is derivable from said source material; and ii) the antigen is administered in a therapeutically effective amount to said individual.
   Alternatively worded, the embodiment relates to an antigen for use in treatment or prophylactic treatment of a hypersensitivity immune response of the gastro-intestinal tract in an individual in need thereof, such as to an antigen for use in bystander suppression of a hypersensitivity immune response of the gastro-intestinal tract in an individual in need thereof, wherein the immune response is triggered by an allergen upon the individual's exposure to a dietary source material comprising said allergen and wherein i) the antigen is unrelated to the allergen(s) triggering the hypersensitivity immune response in the individual and is obtainable from or is derivable from said source material; and ii) the antigen is administered in a therapeutically effective amount to said individual.
III) A method for treatment or prophylactic treatment of a hypersensitivity immune response of the skin (e.g. a hypersensitivity immune response associated with contact dermatitis and/or atopic dermatitis) in an individual in need thereof, such as to a method for bystander suppression of a hypersensitivity immune response of the skin in an individual in need thereof, wherein the immune response is triggered by an allergen upon the individual's exposure to an environmental source material comprising said allergen and wherein; i) the antigen is unrelated to the allergen(s) triggering the hypersensitivity immune response in the individual and is obtainable from or is derivable from said source material; and ii) the antigen is administered in a therapeutically effective amount to said individual.
   Alternatively worded, the embodiment relates to an antigen for use in treatment or prophylactic treatment of a hypersensitivity immune response of the skin (e.g. a hypersensitivity immune response associated with contact dermatitis and/or atopic dermatitis) in an individual in need thereof, such as to an antigen for use in bystander suppression of a hypersensitivity immune response of the skin in an individual in need thereof, wherein the immune response is triggered by an allergen upon the individual's exposure to environmental source material comprising said allergen and wherein i) the antigen is unrelated to the allergen(s) triggering the hypersensitivity immune response in the individual and is obtainable from or is derivable from said source material; and ii) the antigen is administered in a therapeutically effective amount to said individual.

Typical examples of allergen-containing environmental source material and or an allergen-containing dietary source material are an environmental source material (including an airborne environmental source material) or a dietary source material comprising one or more allergen(s) capable of triggering a hypersensitivity immune response in an individual, such as source materials derived from the major taxonomic groups Animalia Arthropoda, Animalia Chordata, Funghi Ascomycetes, Plantae Corniferopsida, Plantae Liliopsida and/or Plantae Magnoliopsida.

More particularly, in interesting embodiments of the invention, the source material is derived from a genus including a species belonging to the order Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales and/or Proteales.

Thus, in interesting embodiments thereof, an antigen of the invention is unrelated to a major allergen or to all allergens of a source material derived from a genus including a species belonging to the order Astigmata, Diptera, Hymenoptera, Carminora, Perissodactyla, Capnodialis; Eurotialis, Hypocreales, Pleosporales and/or Saccharomy.

Accordingly, in various embodiments of the invention, the antigen is unrelated to at least a major allergen, preferably all allergens found in an environmental source material (including an airborne environmental source material) or a dietary source material derived from the major taxonomic groups selected from the group consisting of Animalia Arthropoda, Animalia Chordata, Funghi Ascomycetes, Plantae Corniferopsida, Plantae Liliopsida and Plantae Magnoliopsida. In still various embodiments thereof, the antigen is unrelated to at least a major allergen, preferably all allergens, found in an environmental source material (including an airborne environmental source material) or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales and Proteales.

### Typical examples of source materials and allergens are given below:

In one main embodiment of the invention, the source material is an environmental source material, such as an airborne environmental source material, such as particularly mite crops and mite feces of the major taxonomic group Animalia Arthropoda, dander, hair, saliva, stools or other secretes derived from the major taxonomic group Animalia Chordata; spores or particles derived from the major taxonomic group Funghi Ascomycetes, pollen derived from the major taxonomic group Corniferopsida, pollen derived from the major taxonomic group Plantae Magnoliopsida, pollen derived from the major taxonomic group Plantae Liliae; venoms or secretes derived from the major taxonomic group Animalia Arthropoda; gums or products comprising such a gum derived from trees of the major taxonomic group Plantae Magnoliopsidae.

Still, in one main embodiment of the invention, the source material is a dietary dietary source material, such as a food ingredient or product comprising such a food ingredient, in particularly shrimps or a shrimp-containing food of the major taxonomic group Animalia Arthropoda; lobster or a lobster-containing food of the major taxonomic group Animalia Arthropoda; fruit, legumes, cereals or beans derived from the major taxonomic group Plantae Liliopsidae or a food product comprising such fruits, legumes, cereals and/or beans; fruit, legumes, cereals or beans derived from the major taxonomic group Plantae Magnoliopsidae or a food product comprising such fruits, legumes, cereals and/or beans; nut or a nut-containing food of the from the major taxonomic group Plantae Magnoliopsidae.

Still, in one main embodiment of the invention, the source material is a dietary dietary source material, such as cow's milk, milk-containing food, chicken egg white, chicken egg white-containing food, fish or fish-containing food.

Exemplary source materials derived from crops and feces of the major taxonomic group Animalia Arthropoda are crops and feces derived from the order Astigmata, such as crops and/or feces of a storage mite of the following genus: Acarus (e.g. the species Acarus siro containing at least the allergen Aca s 13); Glycyphagus (e.g. the species Glycyphagus domesticus containing at least the allergen Gly d 2); Lepidoglyphus (e.g. the species (Lepidoglyphus destructor containing at least the one or more allergens Lep d 2, Lep d 5, Lep d 7, Lep d 10 and Lep d 13); of Tyrophagus (e.g. species of Tyrophagus putrescentiae containing at least the one or more allergens Tyr p 2, Tyr p 3, Tyr p 10, Tyr p 13 and Tyr p 24).

Still exemplary source materials thereof are airborne and/or inhalable particles, such as particularly crops and/or feces of a house dust mite of the genus Blomia (e.g. the species Blomia tropicalis (Mite) containing at least the one or more allergens Blo t 1, Blo t 2, Blo t 3, Blo t 4, Blo t 5, Blo t 6, Blo t 10, Blo t 11, Blo t 12, Blo t 13, Blo t 19 and Blo t 21); of the genus Dermatophagoides (e.g. the species Dermatophagoides farinae (American house dust mite) containing at least the one or more allergens Der f 1, Der f 2, Der f 3, Der f 6, Der f 7, Der f 10, Der f 11, Der f 13, Der f 14, Der f 15, Der f 16, Der f 17, Der f 18 and Der f 22; Dermatophagoides microceras (House dust mite) containing at least the allergen Der m 1; Dermatophagoides pteronyssinus (European house dust mite) containing at least the one or more allergens Der p 1, Der p 2, Der p 3, Der p 4, Der p 5, Der p 6, Der p 7, Der p 8, Der p 9, Der p 10, Der p 11, Der p 14, Der p 20, Der p 21 and Der p 23); of the genus Euroglyphus (e.g. Euroglyphus maynei containing at least the one or more allergens Eur m 1, Eur m 2, Eur m 3, Eur m 4 and Eur m 14). Still exemplary source materials thereof are airborne and/or inhalable particles, such as particularly crops and/or feces of a cockroach of the order Blattaria, such as represented by the genus Blatella (e.g. the species Blattella germanica (German cockroach) containing at least the one or more allergens Bla g 1, Bla g 2, Bla g 4, Bla g 5, Bla g 6, Bla g 7 and Bla g 8); and the genus Periplaneta (e.g. the species Periplaneta americana (American cockroach) containing at least the one or more allergens Per a 1, Per a 3, Per a 6, Per a 7, Per a 9 and Per a 10).

Accordingly, in some embodiments of the invention, the airboirne environmental source material is a crop and/or feces of a mite selected from the group consisting of mites of the genus Acarus, Glycyphagus, Lepidoglyphus, Tyrophagus, Blomia, Dermatophagoides, Euroglyphus, Blatella and Periplaneta, such as a crop and/or feces of a mite comprising one or more of the allergens Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9 and Per a 10.

In interesting embodiments thereof, the airborne environmental source material is a crop and/or feces of a storage mite selected from the group consisting of storage mites of the genus Acarus, Glycyphagus, Lepidoglyphus and Tyrophagus, such as a crop and/or feces of a storage mite comprising one or more of the allergens Aca s 13, Gly d 2, Lep d 2, Lep d 5, Lep d 7, Lep d 10 and Lep d 13, Tyr p 2, Tyr p 3, Tyr p 10, Tyr p 13 and Tyr p 24.

In still interesting embodiments thereof, the airborne environmental source material is a crop and/or feces of a house dust mite selected from the group consisting of the genus Blomia, Dermatophagoides and Euroglyphus, such as crop and/or feces of a house dust mite comprising one or more of the allergens Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4 and Eur m 14.

In still interesting embodiments thereof, the airborne environmental source material is a crop and/or feces of a cockroach selected from the group consisting of the genus Blatella and Periplaneta, such as a crop and/or feces of a cockroach comprising one or more of the allergens Bla g 1, Bla g 2, Bla g 4, Bla g 5, Bla g 6, Bla g 7, Bla g 8, Per a 1, Per a 3, Per a 6, Per a 7, Per a 9 and Per a 10.

It should be understood that the airborne environmental source materials in the form of a mite crop and/or mite feces as mentioned above, are of particular relevance in treatment or prophylactic treatment of a hypersensitivity immune reaction of the respiratory tract, in which embodiments, the antigen preferably is unrelated to one or more or all of the allergens selected from the group consisting of Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9 and Per a 10, and preferably wherein the antigen is obtainable from or derivable from said crop and/or feces of said genus of mite.

Still further examples of environmental source materials including airborne environmental source materials are materials, such as dander, hair, saliva, stools or other secretes, derived from the major taxonomic group Animalia Chordata. Representative examples of such materials are derived from genus belonging to the order Carminora, such as the genus Canis (e.g. the species Canis familiaris (dog) containing at least the one or more allergens Can f 1, Can f 2, Can f 3, Can f 4, Can f 5 and Can f 6); the genus Felis (e.g. the species Felis domesticus (cat) containing at least the one or more allergens Fel d 1, Fel d 2, Fel d 3, Fel d 4, Fel d 5w, Fel d 6w, Fel d 7 and Fel d 8. Further representative examples thereof are materials, such as dander, hair, saliva stools or other secretes of species belonging to the order Perissodactyla, such as the genus Equus (e.g. the species Equus caballus (domestic horse) containing at least the one or more allergens Equ c 1, Equ c 2, Equ c 3, Equ c 4 and Equ c 5).

Accordingly, in some embodiments of the invention, the environmental source material including an airborne material thereof is dander, hair, saliva or stools of an animal of the genus canis, felis or Equus, such as dander, hair, saliva or stool comprising one or more of the allergens Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5.

Thus, it should be understood that in interesting embodiments, the invention relates to treatment or prophylactic treatment of a hypersensitivity immune reaction of the respiratory tract in an individual in need thereof, wherein the immune response is triggered by an allergen upon the individual's exposure to hair, dander, saliva or stool of an animal of the taxonomic group Animalia Arthropoda (e.g. of the genus canis, felis or Equus) comprising said allergen(s); and preferably wherein the antigen is unrelated to the allergen triggering the hypersensitivity immune response in the individual (such as to one or more or all of the allergens selected from the group consisting of Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5); and preferably wherein the antigen is obtainable from or is derivable from said hair, dander, saliva or stool of said genus of an animal.

Still further examples of environmental source materials including airborne materials thereof are spores or particles derived from the major taxonomic group Funghi Ascomycetes. Typical examples of such materials are derived from the genus belonging to the order Capnodialis, such as the genus Cladosporium (e.g. the species Cladosporium cladosporioides containing at least the one or more allergens Cla c 9 and Cla c 14; Cladosporium herbarum containing at least the one or more allergens Cla h 2, Cla h 5, Cla h 6, Cla h 7, Cla h 8, Cla h 9, Cla h 10 and Cla h 12). Further examples thereof are genus belonging to the order Eurotialis, such as the genus Aspergillus (e.g. the species aspergillus flavus (fungus) containing at least the allergen Asp fl 13; Aspergillus fumigatus (fungus) containing at least the one or more allergens Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29 and Asp f 34; Aspergillus niger containing at least the one or more allergens Asp n 14, Asp n 18 and Asp n 25; Aspergillus oryzae containing at least the one or more allergens Asp o 13 and Asp o 21; Aspergillus versicolor containing at least the allergen Asp v 13); such as the genus Penicillium (e.g. the species Penicillium brevicompactum containing at least the one or more allergens Pen b 13 and Pen b 26; Penicillium chrysogenum containing at least the one or more allergens Pen ch 13, Pen ch 18, Pen ch 20, Pen ch 31, Pen ch 33 and Pen ch 35; Penicillium citrinum containing at least the one or more allergens Pen c 3, Pen c 13, Pen c 19, Pen c 22, Pen c 24; Pen c 30 and Pen c 32; and Penicillium oxalicum containing at least the allergen Pen o 18). Further examples thereof are genus belonging to the order Hypocreales, such as the genus Fusarium (e.g. the species Fusarium culmorum (N.A.) containing at least the one or more allergens Fus c 1 and Fus c 2; Stachybotrys chartarum containing at least the allergen Sta c 3). Still further examples thereof are genus belonging to the order Pleosporales, such as the genus Alternaria (e.g. the species Alternaria alternata (Alternaria rot fungus) containing at least the one or more allergens Alt a 1, Alt a 3, Alt a 4, Alt a 5, Alt a 6, Alt a 7, Alt a 8, Alt a 10, Alt a 12 and Alt a 13). Further examples thereof are genus belonging to the order Saccharomycetes, such as the genus Candida (e.g. of the species Candida albicans (Yeast) containing at least the one or more allergens Cand a 1 and Cand a 3 and Candida boidinii (Yeast) containing at least the allergen Cand b 2.

Accordingly, in some embodiments of the invention, the source material is derived from the genus Cladosporium, Aspergillus, Penicillium, Alternaria and Candida, such as a yeast, mould or funghi of the genus and Cladosporium, Aspergillus, Penicillium, Alternaria and Candida. It follows that in some embodiments of the invention, the source material is a yeast, mould or funghi comprising one or more of the allergens Cla c 9m Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2.

Accordingly, it should be understood that in interesting embodiments, treatment of or prophylactic treatment of a hypersensitivity immune response of the respiratory tract in an individual in need thereof, wherein the immune response is triggered by an allergen upon the individual's exposure to yeast, mould or funghi of the genus selected from the group consisting of Cladosporium, Aspergillus, Penicillium, Alternaria or Candida; preferably wherein the antigen is unrelated to the allergen triggering the hypersensitivity immune response in the individual (such as unrelated to one or more or all of the allergens selected from the group comprising Cla c 9m Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2), preferably, wherein the unrelated antigen is obtainable from or is derivable from said genus of yeast, mould or funghi.

Still further examples of environmental source materials including airborne materials thereof are materials derived from plants and trees, such as airborne particles and/or inhalable particles, such as particularly pollen derived from the major taxonomic group Corniferopsida. Representative examples of materials belonging this group are genus belonging to the order Corniferales, such as the genus Chamaecyparis (e.g. the species Chamaecyparis obtusa (Japanese cypress) containing at least the one or more allergens Cha o 1 and Cha o 2); such as the genus Cryptomeria (e.g. the species Cryptomeria japonica (Sugi) containing at least the one or more allergens Cry j 1 and Cry j 2); such as the genus Cupressus (e.g. the species Cupressus arizonica Cypress) containing at least the allergen Cup a 1; Cupressus sempervirens (Common cypress) containing at least the one or more allergens Cup s 1 and Cup s 3); such as of the genus Juniperus (e.g. the species Juniperus ashei (Mountain cedar) containing at least the one or more allergens Jun a 1, Jun a 2 and Jun a 3; Juniperus oxycedrus (Prickly juniper) containing at least the allergen Jun o 4; Juniperus sabinoides (Mountain cedar) containing at least the allergen Jun s 1 and Juniperus virginiana (Eastern red cedar) containing at least the one or more allergens Jun v 1 and Jun v 3).

Still further examples of environmental source materials are materials of plants and trees, such as airborne particles and/or inhalable particles, such as particularly pollen derived from the major taxonomic group Plantae Liliopsida. Representative examples of genus belonging to this group are genus belonging to the order Poales, such as the genus Anthoxanthum (e.g the species Anthoxanthum odoratum (Sweet vernal grass) containing at least the allergen Ant o 1); of the genus Cynodon (e.g. the species Cynodon dactylon (Bermuda grass) containing at least one or more of the allergens Cyn d 1, Cyn d 7, Cyn d 12, Cyn d 15, Cyn d 22w, Cyn d 23 and Cyn d 24; such as the genus Dactylic (e.g. the species Dactylis glomerata (Orchard grass) containing at least one or more of the allergens Dac g 1, Dac g 2, Dac g 3, Dac g 4, and Dac g 5); such as of the genus Festuca (e.g. the species Festuca pratensis (Meadow fescue) containing at least the allergen Fes p 4; such as of the genus Holcus (e.g. the species Holcus lanatus (Velvet grass) containing at least one or more of the allergens Hol l 1 and Hol l 5); such as of the genus Hordeum (e.g. the species Hordeum vulgare (Barley) containing at least one or more of the allergens Hor v 1 and Hor v 5); such as of the genus Lolium (e.g. the species Lolium perenne (Rye grass) containing at least one or more of the allergens Lol p 1, Lol p 2, Lol p 3, Lol p 4, Lol p 5 and Lol p 11); such as of th genus Oryza (e.g. the species Oryza sativa (Rice) containing at least the allergen Ory s 1); such as of the genus Phleum (e.g. the species Phleum pratense (Timothy) containing at least one or more of the allergens Phl p 1, Phl p 2, Phl p 4, Phl p 5, Phl p 6, Phl p 7, Phl p 11, Phl p 12 and Phl p 13); such as of the genus Poa (e.g. the species Poa pratensis (Kentucky blue grass) containing at least one or more of the allergens Poa p 1 and Poa p 5); such as of the genus Secale (e.g. the species Secale cereale (Rye) containing at least one or more of the allergens Sec c 1, Sec c 5 and Sec c 20); such as of the genus Sorghum (e.g. the species Sorghum halepense (Johnson grass) containing at least the allergen Sor h 1); such as of the genus Triticum (e.g. the Triticum aestivum (Wheat) containing at least one or more of the allergens (Tri a 15, Tri a 21, Tri a 27, Tri a 28, Tri a 29, Tri a 30, Tri a 31, Tri a 32, Tri a 33, Tri a 34 and Tri a 35); and such as of the genus Zea (e.g. the species Zea mays (Maize) containing at least one or more of the allergens Zea m 1 and Zea m 12).

Further examples of environmental source materials are materials of plants and trees, such as airborne particles and/or inhalable particles, such as particularly pollen derived from the major taxonomic group Plantae Magnoliopsida. Representative examples of this group are genus belonging to the order Asterales, such as the genus Ambrosia (e.g. the species Ambrosia artemisiifolia (Short ragweed) containing at least one or more of the allergens Amb a 1, Amb a 2, Amb a 3, Amb a 4, Amb a 5, Amb a 6, Amb a 7, Amb a 8, Amb a 9 and Amb a 10; Ambrosia psilostachya (Western ragweed) containing at least allergens Amb p 5; Ambrosia trifida (Giant ragweed) containing at least the allergen Amb t 5); such as of the genus Artemisia (e.g. the species Artemisia vulgaris (Mugwort) containing at least one or more of the allergens Art v 1, Art v 2, Art v 3, Art v 4, Art v 5 and Art v 6) and such of the genus Helanthus (e.g. the species Helianthus annuus (Sunflower) containing at least one or more of the allergens Hel a 1, Hel a 2 and Hel a 3. Still representative examples of this group are genus belonging to the order Fagales, such as the genus Alnus (e.g. Alnus glutinosa (Alder) containing at least one or more of the allergens Aln g 1 and Aln g 4); such as of the genus Betula (e.g. the species Betula verrucosa (Birch) containing at least one or more of the allergens Bet v 1, Bet v 2, Bet v 3, Bet v 4, Bet v 6 and Bet v 7); such as of the genus Corylus (e.g. Corylus avellana (Hazel) containing at least the allergen Cor a 10); Still representative examples of this group are genus belonging to the order Lamiales, such as the genus Fraxius (e.g. the species Fraxinus excelsior (Ash) containing at least the allergen Fra e 1; such as the genus Olea (e.g. the species Olea europea (Olive) containing at least one or more of the allergens Ole e 1, Ole e 2, Ole e 3, Ole e 4, Ole e 5, Ole e 6, Ole e 7, Ole e 8, Ole e 9, Ole e 10 and Ole e 11; Still representative examples of this group are genus belonging to the order Proteales, such as the genus Platanus (e.g. the species Platanus acerifolia (London plane tree) containing at least one or more of the allergens Pla a 1, Pla a 2 and Pla a 3 and Platanus orientalis (Oriental plane) containing at least one or more of the allergens Pla or 1, Pla or 2 and Pla or 3).

Thus, in still interesting embodiments of the invention, the source material is a pollen of the major taxonomic group Corniferopsida, Plantae Liliopsida or Plantae Magnoliopsida, such as a pollen of the genus selected from the group comprising Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea and Platanus.

That is to say that in still interesting embodiments of the invention, the source material is a pollen comprising one or more of the allergen(s) Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1 Zea m 12, Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3.

Accordingly, one aspect of the invention relates to treatment of or prophylactic treatment of a hypersensitivity immune response of the respiratory tract in an individual in need thereof, wherein the immune response is triggered by an allergen upon the individual's exposure to pollen of the major taxonomic group Corniferopsida, Plantae Liliopsida or Plantae Magnoliopsida (such as a pollen of the genus selected from the group comprising Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea and Platanus comprising said allergen(s); preferably wherein the antigen is unrelated to the allergen triggering the hypersensitivity immune response in the individual (such as to one or more or all of the allergens selected from the group comprising of Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1 Zea m 12, Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3; and preferably wherein the unrelated antigen is obtainable from or is derivable from said pollen.

In preferable embodiments thereof the source material is a grass pollen of the genus selected from the group consisting of Anthoxanthum, Dactylis, Lolium, Phleum and Poa, such as a grass pollen comprising one or more of the allergen(s) Ant o 1, Dac g 1, Dac g 2, Dac g 3, Dac g 4, Dac g 5, Lol p 1, Lol p 2, Lol p 3, Lol p 4, Lol p 5, Lol p 11, Phl p 1, Phl p 2, Phl p 4, Phl p 5, Phl p 6, Phl p 7, Phl p 11, Phl p 12, Phl p 13, Poa p 1 and Poa p 5.

In still preferable embodiments thereof the source material is a weed pollen of the genus selected from the group consisting of Cupressus, Juniperus, Alnus, Betula and Olea, such as a tree pollen comprising one or more of the allergen(s) Cup a 1, Cup s 1, Cup s 3, Jun a 1, Jun a 2, Jun a 3, Jun o 4, Jun s 1, Jun v 1 and Jun v 3, Aln g 1, Aln g 4, Bet v 1, Bet v 2, Bet v 3, Bet v 4, Bet v 6, Bet v 7, Ole e 1, Ole e 2, Ole e 3, Ole e 4, Ole e 5, Ole e 6, Ole e 7, Ole e 8, Ole e 9, Ole e 10 and Ole e 11.

In still preferable embodiments thereof the source material is a tree pollen of the genus selected from the group consisting of Ambrosia and Artemisia, such as a weed pollen comprising one or more of the allergen(s) Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5 and Art v 6.

Still further typical examples of environmental source materials are materials, such as venoms, saliva, or other secretes, derived from the major taxonomic group Animalia Arthropoda. Representative examples thereof are materials derived from the order Diptera, such as a mosquito of the genus Aedes (e.g. the species Aedes aegypti (Yellow fever mosquito) containing at least the one or more allergens Aed a 1, Aed a 2 and Aed a 3), such as a midge of the genus Chironomus (e.g. Chironomus kiiensis containing at least the allergen Chi k 10 and Chironomus thummi thummi containing at least the one or more allergens Chi t 1, Chi t 2, Chi t 3, Chi t 4, Chi t 5, Chi t 6, Chi t 7, Chi t 8, and Chi t 9). Still further representative examples thereof are venoms, saliva or other secretes of genus and species belonging to the order Hymenoptera, such as a bee of the genus Apis (e.g. Apis cerana (Eastern hive bee) containing at least the allergen Api c 1; Apis dorsata (Giant honeybee) containing at least the allergen Api d 1; Apis mellifera (Honey bee) containing at least the one or more allergens Api m 1, Api m 2, Api m 3, Api m 4, Api m 5, Api m 6, Api m 7, Api m 8, Api m 9, Api m 10 and Api m 11) of the genus Bombus (e.g. the species Bombus pennsylvanicus (Bumble bee) containing at least the one or more allergens Bom p 1 and Bom p 4; Bombus terrestris (Bumble bee) containing at least the one or more allergens Bom t 1 and Bom t 4); of the genus Dolichovespula (e.g. the species Dolichovespula arenaria (Yellow hornet) containing at least the allergen Dol a 5; Dolichovespula maculata (White face hornet) containing at least the one or more allergens Dol m 1, Dol m 2 and Dol m 5); such as the genus Polistes (e.g. the species Polistes annularis (Wasp) containing at least the one or more allergens Pol a 1, Pol a 2 and Pol a 5; Polistes dominulus (Mediterranean paper wasp) containing at least the one or more allergens Pol d 1, Pol d 4 and Pol d 5; Polistes exclamans (Wasp) containing at least the one or more allergens Pol e 1, Pol e 4 and Pol e 5; Polistes fuscatus (Wasp) containing at least the allergen Pol f 5; Polistes gallicus (Wasp) containing at least the one or more allergens Pol g 1 and Pol g 5; Polistes metricus (Wasp) containing at least the allergen Pol m 5); such as of the genus Polybia (e.g. the species Polybia paulista (Wasp) containing at least the allergen Poly p 1; Polybia scutellaris (Wasp) containing at least the allergen Poly s 5); such as of the genus Vespa (e.g. the species Vespa crabro (European hornet) containing at least the one or more allergens Vesp c 1 and Vesp c 5; Vespa magnifica (Hornet) containing at least the one or more allergens Vesp ma 2 and Vesp ma 5; Vespa mandarinia (Giant asian hornet) containing at least the one or more allergens Vesp m 1 and Vesp m 5), such as of the genus Vespula (e.g. the species Vespula flavopilosa (Yellow jacket) containing at least the allergen Ves f 5; Vespula germanica (Yellow jacket) containing at least the allergen Ves g 5; Vespula maculifrons (Yellow jacket) containing at least the one or more allergens Ves m 1, Ves m 2, and Ves m 5; Vespula pensylvanica (Yellow jacket) containing at least the allergen Ves p 5; Vespula squamosa (Yellow jacket) containing at least the one or more allergens Ves s 1 and Ves s 5; Vespula vidua (Wasp) containing at least the allergen Ves vi 5; and Vespula vulgaris (Yellow jacket) containing at least the one or more allergens Ves v 1, Ves v 2, Ves v 3 and Ves v 5.

Accordingly, in some embodiments of the invention, the environmental source material is venom and/or saliva and/or another secrete of a bee of the genus Apis, Bombus Dolichovespula, Polistes, Polybia, Vespa and Vespula. Such as a source material in the form of venom and/or a saliva and/or another secrete of a bee comprising one or more of the allergens Api c 1, Api d 1; Api m 1, Api m 2, Api m 3, Api m 4, Api m 5, Api m 6, Api m 7, Api m 8, Api m 9, Api m 10, Api m 11, Bom p 1, Bom p 4; Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5; Pol g 1, Pol g 5, Poly p 1, Poly s 5; Vesp c 1, Vesp c 5, Vesp ma 2, Vesp ma 5, Vesp m 1, Vesp m 5, Ves g 5, Ves m 1, Ves m 2, Ves m 5; Ves p 5; Ves s 1, Ves s 5; Ves vi 5; Ves v 1, Ves v 2, Ves v 3 and Ves v 5.

Thus, it should be understood that in interesting embodiments, the invention relates to treatment or prophylactic treatment of a hypersensitivity immune response of the skin and/or anaphylaxis in an individual in need thereof, wherein the immune response is triggered an allergen upon the individual's exposure to venom of the taxonomic group Animalia Arthropoda (genus Apis, Bombus Dolichovespula, Polistes, Polybia, Vespa and Vespula) comprising said allergen(s) and wherein the antigen is unrelated to the allergen triggering the hypersensitivity immune response in the individual (such as to one or more or all of the allergens selected from the group consisting of Api c 1, Api d 1; Api m 1, Api m 2, Api m 3, Api m 4, Api m 5, Api m 6, Api m 7, Api m 8, Api m 9, Api m 10, Api m 11, Bom p 1, Bom p 4; Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5; Pol g 1, Pol g 5, Poly p 1, Poly s 5; Vesp c 1, Vesp c 5, Vesp ma 2, Vesp ma 5, Vesp m 1, Vesp m 5, Ves g 5, Ves m 1, Ves m 2, Ves m 5; Ves p 5; Ves s 1, Ves s 5; Ves vi 5; Ves v 1, Ves v 2, Ves v 3 and Ves v 5) and obtainable from or derivable from said bee venom of said genus of bee.

Still further examples of environmental source materials are materials, such as particularly gums or products comprising such a gum, derived from trees of the major taxonomic group Plantae Magnoliopsidae in particularly of a genus belonging to the order Malpighiales, such as the genus Hevea (e.g. the species Hevea brasiliensis (Para rubber tree (latex) containing at least one or more of the allergens Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14.

Thus, it should be understood that in interesting embodiments, the invention relates to treatment or prophylactic treatment of a hypersensitivity immune response of the skin in an individual in need thereof, wherein the immune response is triggered by a gum or products comprising such a gum derived from the genus Hevea comprising said allergen(s), preferably wherein the antigen is unrelated to the allergen triggering the hypersensitivity immune response in the individual (e.g. unrelated to at least one or more of the allergens containing at least one or more of the allergens Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14; preferably wherein the unrelated antigen is obtainable from or is derivable from said gum of said genus.

Typical examples of a dietary source material are food ingredients derived from the major taxonomic group Animalia Arthropoda or any food comprising such an ingredient. Representative examples are food ingredients or foods derived from a genus belonging to the order Decapoda, such as a shrimp or a shrimp-containing food, wherein the shrimp is of the genus Artemia (e.g. the species Artemia franciscana (Brine shrimp) containing at least the allergen Art fr 5; of the genus Crangon (e.g. the species Crangon crangon (North Sea shrimp) containing at least one or more of the allergens Cra c 1, Cra c 2, Cra c 4, Cra c 5, Cra c 6 and Cra c 8); of the genus Litopenaeus (e.g. the species Litopenaeus vannamei (White shrimp) containing at least one or more of the allergens Lit v 1, Lit v 2, Lit v 3 and Lit v 4); of the genus Metapenaeus (e.g. the Metapenaeus ensis (Shrimp) containing at least the allergen Met e 1); of the genus Penaeus (e.g. the species Penaeus aztecus (Shrimp) containing at least the allergen Pen a 1; Penaeus indicus (Shrimp) containing at least the allergen Pen i 1; Penaeus monodon (Black tiger shrimp) containing at least one or more of the allergens Pen m 1, Pen m 2, Pen m 3, Pen m 4 and Pen m 6). Further representative examples are a lobster or a lobster-containing food of the genus Homarus (e.g. the species Homarus americanus (American lobster) containing at least one or more of the allergens Hom a 1, Hom a 3 and Hom a 6); such as of the genus Panulirus (e.g. the species Panulirus stimpsoni (Spiny lobster) containing at least the allergen Pan s 1).

Still typical examples of a dietary source material are food ingredients derived from the major taxonomic group Plantae Liliopsidae or any food comprising such an ingredient. Typical examples of a dietary source material are a cereal or a cereal-containing food of the major taxonomic group Plantae Liliopsidae, such as of a genus belonging to the order Poales, such as the genus Hordeum (e.g the species Hordeum vulgare (Barley) containing at least one or more of the allergens Hor v 12, Hor v 15, Hor v 16, Hor v 17 and Hor v 21); such as of the genus Oryza (e.g. the species Oryza sativa (Rice) containing at least the allergen Ory s 12); such as of the genus Secale (e.g. the species Secale cereale (Rye) containing at least the allergens Sec c 20); such as of the genus Triticum (e.g. the species Triticum aestivum (Wheat) containing at least one or more of the allergens Tri a 12, Tri a 14, Tri a 18, Tri a 19, Tri a 21, Tri a 25, Tri a 26 and Tri a 36); such as of the genus Zea (e.g the species Zea mays (Maize) containing at least one or more of the allergens Zea m 14 and Zea m 25).

Still typical examples of a dietary source material are food ingredients derived from the major taxonomic group Plantae Magnoliopsida or any food comprising such an ingredient. Still typical examples of a dietary source material are a nut or a nut-containing food of a genus belonging to the order Fabales, such as the genus Arachis (e.g. the species Arachis hypogaea (Peanut) containing at least one or more of the allergens Ara h 1, Ara h 2, Ara h 3, Ara h 4, Ara h 5, Ara h 6, Ara h 7, Ara h 8, Ara h 9, Ara h 10 and Ara h 11) of the order Fagales, such as the genus Corylis (e.g. the species Corylus avellana (Hazel) containing at least one or more of the allergens Cor a 1, Cor a 2, Cor a 8, Cor a 9, Cor a 11, Cor a 12, Cor a 13 and Cor a 14); such as of the genus Juglans (e.g. the species Juglans nigra (Black walnut) containing at least one or more of the allergens Jug n 1 and Jug n 2; Juglans regia (English walnut) containing at least one or more of the allergens Jug r 1, Jug r 2, Jug r 3, Jug r 4). Still more representative examples are nuts or nut-containing foods of the order rosales, such as the genus Prunus (e.g. the species Prunus dulcis (Almond) containing at least one or more of the allergens Pru du 3, Pru du 4, Pru du 5 and Pru du 6). Still more representative examples are nuts or nut-containing foods of the order sapindales, such as of the genus Anacardium (e.g .the species Anacardium occidentale (Cashew) containing at least one or more of the allergens Ana o 1, Ana o 2 and Ana o 3;, such as of the genus Pistacia (e.g. the species Pistacia vera (Pistachio) containing at least one or more of the allergens Pis v 1, Pis v 2, Pis v 3, Pis v 4 and Pis v 5.

Still typical examples of a dietary source material are food ingredients derived from the major taxonomic group Plantae Magnoliopsidae or any food comprising such an ingredient. Examples are beans or bean-containing food of a genus belonging to the order Fabales, such as the genus Glycine (e.g. the species Glycine max (Soybean) containing at least one or more of the allergens Gly m 1, Gly m 2, Gly m 3, Gly m 4, Gly m 5 and, Gly m 6).

Still typical examples of a dietary source material is cow's milk or milk-containing food, such as a a dietary source material comprising an allergen of cow's milk, such as a casein and/or a beta-lacto-globulin of cow's milk.

Still typical examples of a dietary source material is chicken egg white or chicken egg white-containing food, such as a a dietary source material comprising an ovalbumin, ovomucoid of chicken egg white.

Still typical examples of a dietary source material are a fish or fish-containing food, such as a dietary source material comprising allergen M of a fish.

Therefore, in interesting embodiments of the invention, the dietary source material is a food ingredient including a food comprising the food ingredient, wherein the food ingredient is derived from a genus selected from Hordeum, Oryza, Secale, Triticum, Zea, Arachis, Corylis, Juglans, Prunus, Anacardium, Pistacia and Glycine.

That is to say that in still interesting embodiments of the invention, the dietary source material is a food ingredient including a food comprising the food ingredient, wherein the food ingredient comprises one or more of the allergen(s) Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and, Gly m 6.

In still representative embodiments, the dietary source material is cow's milk, milk-containing food, chicken egg white, chicken egg white-containing food, fish or fish-containing food, such as a dietary source material comprising one or more or all of the allergens casein of cow's milk, beta-lacto-globulin of cow's milk, ovalbumin of chicken egg white, ovomucoid of chicken egg white and allergen M of a fish.

In preferable embodiments thereof the dietary source material is a cereal or a cereal-containing food of the genus selected from the group consisting of Hordeum, Oryza, Secale, Triticum and Zea, such as a a cereal or a cereal-containing food comprising one or more of the allergen(s) Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14 and Zea m 25.

In still preferable embodiments thereof the source material is a nut or a nut-containing food of the genus selected from the group consisting of Arachis, Corylis, Juglans, Prunus, Anacardium and Pistacia, such as nut or a nut-containing food comprising one or more of the allergen(s) Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4 and Pis v 5.

In still preferable embodiments thereof the source material is a bean or a bean containing food of the genus Glycine, such as a bean or bean-containing food comprising one or more of the allergen(s) Gly m 1, Gly m 2, Gly m 3, Gly m 4, Gly m 5 and, Gly m 6.

It should be understood that in interesting embodiments, the invention relates to treatment or prophylactic treatment of a hypersensitivity immune response of the gastro-intestinal tract in an individual in need thereof, wherein the immune response is triggered by an allergen of a dietary source material comprising said allergen(s), preferably wherein the dietary source material derived from, such as a food ingredient or product comprising the food ingredient derived from, a genus selected from Hordeum, Oryza, Secale, Triticum, Zea, Arachis, Corylis, Juglans, Prunus, Anacardium, Pistacia and Glycine; preferably wherein the antigen is unrelated to the allergen triggering the hypersensitivity immune response in the individual (e.g. one or more or all of the allergens Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and/or, Gly m 6; preferably wherein the unrelated antigen is obtainable from or is derivable from said dietary source material.

Also, in still interesting embodiments, the invention relates to treatment or prophylactic treatment of a hypersensitivity immune response of the gastro-intestinal tract in an individual in need thereof, wherein the immune response is triggered by an allergen of a dietary source material comprising said allergen(s), preferably wherein the dietary source material is derived from cow's milk, milk-containing food, chicken egg white, chicken egg white-containing food, fish or fish-containing food; preferably wherein the antigen is unrelated to the allergen triggering the hypersensitivity immune response in the individual (e.g. one or more or all of the allergens one or more or all of the allergens casein of cow's milk, beta-lacto-globulin of cow's milk, ovalbumin of chicken egg white, ovomucoid of chicken egg white and/or allergen M of a fish; preferably wherein the unrelated antigen is obtainable from or is derivable from said dietary source material.

It might be summarized that typical examples of environmental source materials originating from pollen are, but not limited to
- plant and tree pollen of various species of the genus Ambrosia (ragweeds); of the genus Alder (Alnus); of the genus Artemisia; of the genus Betula (Birch); of the genus Dactylis (Orchard Grass); of the genus Quercus (Oaks); of the genus Olea (Olive Oil Trees); of the genus Parietaria (weeds); of the genus Cupressus (Redwoods)); of the genus Juniperus (Cedar trees); Festuca (Fescue grasses); of the genus Avena; of the genus Holcus; of the genus Anthoxanthum; of the genus Arrhenatherum; of the genus Agrostis; of the genus Phleum (Timothy grasses); of the genus Poa (Blue Grass); of the genus Cynodon (Bermuda Grass) of the genus Phalaris; of the genus Paspalum; of the genus Lolium (Rye grass).

Typical examples of environmental source materials of animal origin are, but not limited to;
- dander, hair, urine and faeces of the genus canis, of the family felis, of the genus Equus, of the genus Periplanbeta (American cockroach), of the genus Blomia (storage mite) and of the genus Dermatophagoides;

Typical examples of environmental source materials of fungal spores, yeast and moulds are, but not limited to - fungal spores, yeast and moulds of various species from the genus Alternaria, Aspergillus and Cladosporium;

Still other examples of environmental source material are venoms derived from insects, such as venoms from the class of insects named hymenoptera, more specifically from the insects of the family named Apidae (such as honey bees and bumble bees) and the family named Vespidae (such as wasps (the Vespula species), hornets, and paper wasps).

In particular interesting embodiments, the environmental source material is pollen of various species of the genus Ambrosia (ragweeds); of the genus Alder (Alnus); of the genus Betula (Birch); of the genus Olea (Olive Oil Tree); of the genus Cupressus (Redwoods); of the genus Juniperus¨(Cedar) and of the genus Phleum.

As mentioned, a source material of the invention may also be a dietary inclusive foods, feeds, drinks and industrial produced dietary's. Typical examples, on such source materials are, but not limited to, various species of nuts, such as of the tree nuts including macadamia nuts, brazil nuts, cashews, almonds, walnuts, pecans, pistachios, chestnuts, beechnuts, hazelnuts, pine nuts, gingko nuts and hickory nuts as well as industrial dietary products therof.

Other food sources are cereals containing gluten (i.e. wheat, rye, barley, oats, spelt); fish; crustaceans; egg; peanut; soybeans (soy protein, textured vegetable protein TPV, hydrolysed plant protein, hydrolysed soy protein, hydrolysed vegetable protein); milk and dairy products including lactose (milk sugar); nuts (e.g. almond (Amygdalus communis), hazelnut (Corylus avellana), walnut (Juglans regia), cashew (Anacardium occidentale), pecan nut(Carya illinoiesis), Brazil nut (Bertholletia excelsa), pistachio nut (Pistacia vera), macadamia nut, Queensland nut (Macadamia ternifolia)); celery and other foods of the Umbelliferae family; mustard; and sesame seed as well as industrial dietary products thereof.

### Treatment and administration of unrelated antigen

As mentioned, the antigen of the invention is used in treatment of a hypersensitivity immune response. More particularly, the hypersensitivity immune response is associated with an allergic disease/allergic immune response, such as a type 1 or a type 4 hypersensitivity immune response. Notably, it should be understood that the method not necessarily encompass the treatment of an individual so as to prevent sensitization to another allergen than the one(s) an individual already is sensitized to, such as for example to precent epicutaneous sensitization with a new protein allergen.

Typical examples of diseases mediated by a hypersensitivity immune response are, but not limited to, allergic diseases like atopic dermatitis, urticaria, contact dermatitis, allergic conjunctivitis, allergic rhinitis, allergic asthma, anapylaxis, food allergy, drug allergy.

Typical examples of diseases mediated by a type 1 hypersensitivity immune reaction are, but not limited to, allergic diseases like atopic dermatitis, allergic conjunctivitis, allergic rhinitis, allergic asthma, anapylaxis, food allergy, drug allergy and hay fever.

In particularly, a hypersensitivity immune response of the respiratory tract is associated to allergic rhinitis and/or allergic asthma; a hypersensitivity immune response of the gastro-intestinal tract is associated to food allergy and a hypersensitivity immune response of the skin is associated to contact dermatitis and/or atopic dermatitis.

It is anticipated that the administration to mucosa of the oral cavity may be performed by topically delivering the antigen at the surface of an epithelia of the oral cavity from where the antigen is absorbed into the mucosa and sub mucosa. For example, the antigen is administered to the epithelia of the sublingual part including the floor of the mouth from where the antigen is absorbed into the mucosa and sub mucosa. It should be understood that in more interesting embodiments of the invention, the antigen is administered by sublingual administration.

In particularly, a hypersensitivity immune response of the respiratory tract is associated to allergic rhinitis and/or allergic asthma; a hypersensitivity immune response of the gastro-intestinal tract is associated to food allergy and a hypersensitivity immune response of the skin is associated to contact dermatitis and/or atopic dermatitis.

It is anticipated that an antigen of the invention may be administered to epithelia, mucosa or to skin. For example, it might be important to administrate the antigen by a route assisting the delivery of the antigen to an antigen-presenting cell or to lymph nodes. The administration to an epithelia may comprise the administration of the antigen topically to epithelia of the skin, oral cavity and/or the gastro-intestinal tract, whereas mucosal administration generally include administration to the mucosa of the oral cavity, gingiva, sublingual part, gastric, intestinal, nasal or lung mucosa. Alternatively, the antigen is administered directly to a lymph node. Mucosal administration may be performed by topically delivering the antigen at the surface of epithelia from where the antigen is absorbed into the mucosa and submucosa. Thus, mucosal administration may include delivering the surface of epithelia of the oral cavity including the sublingual part, gastro-intestinal tract, respiratory tract, nasal cavity or eye cavity.

As found by the present inventors the administration of the unrelated antigen to a mucosa of the oral cavity, such as by the sublingual route of administration, is an accurate, convenient and liable administration route providing higher bystander suppression of a hypersensitivity immune response than via per-oral administration of the same antigen.

Therefore, in still further embodiments of the invention, the antigen of the invention is administered by sublingual administration, i.e. topically to the sublingual epitelia or directly to the sublingual mucosa. As the sublingual route forms part of the oral cavity, it is also preferred to administer the unrelated antigen to the epithelia or mucosa of the oral cavity, such as by buccal administration. By buccal administration it is the intention to apply a suitable pharmaceutical formulation of the unrelated antigen on a mucosa of the oral cavity, such as the sublingual mucosa, at the upper surface of the tongue, at the gum or at the cheeks, so as to deliver the unrelated antigen to the mucosa of the oral cavity and wherein it is not the intention to deliver the unrelated antigen to the gastro-intestinal tract. Thus, in more preferable embodiments of the invention, the antigen is administered to the mucosa of the oral cavity, such as by sublingual administration, but where the individual is instructed not to swallow the unrelated antigen before a period of at least 1 minute after the administration of the unrelated antigen, such as more preferably after 2 minutes, 3 minutes, 4 minutes, or 5 minutes of administration of the unrelated antigen.

As mentioned, the unrelated antigen of the invention might be used in the treatment of a hypersensitivity immune reaction. The dosage regimen of relevance might be one usually applied in the field of allergen specific immunotherapy, for example, in terms of selecting doses, number of doses per day, duration of treatment and frequency of administration. Thus, it might be envisaged that the unrelated antigen be administered frequently during a longer period before the desirable effect is achieved, such as administered daily to the mucosa of the oral cavity (e.g. sublingual mucosa) for a period of at least 6 months. Also it is envisaged that where the hypersensitivity immune response is caused by a seasonal allergen, the first dose may be administered before the allergen season. It is also envisaged that the treatment is initiated by an up-dosing phase where the unrelated antigen is administered in increasingly doses during one day or with days between until a maintenance dose is achieved.

It should be understood that the unrelated antigen is the only antigen administered in the present treatment regiment to the oral cavity. In particularly, the allergen to which the individual has specific IgE antibodies towards shall not be co-administered with the unrelated antigen. Most suitable an allergen is not co-administered with the unrelated antigen at all, such as not co-administered with the unrelated antigen to the oral cavity. It should also be understood that the unrelated antigen is not administered together with a modulator of the Notch signalling pathway either as a combined preparation for simultaneous, contemporaneous, separate or sequential use for modulation of the immune system as described in WO 2004/082710.

### Co-exposure/Co-availability by administering unrelated antigen to target organ

As mentioned, it is crucial to the invention, that the individual be co-exposed to the unrelated antigen, at least at the time the individual is exposed to an allergen-containing source material comprising the allergen triggering the hypersensitivity immune response in the individual.

Accordingly, the unrelated antigen is also administered to the target organ, such as the respiratory tract, gastro-intestinal tract, or skin within a period at least coinciding partly or entirely with the individual's exposure to said source material.

The phrase "the antigen is also administered ....within a period at least coinciding partly or entirely with the individual's exposure to a source material" is meant to designate that the unrelated antigen is administered to the target organ that is exposed to the "triggering" allergen and that the administration of the unrelated antigen shall be performed at least during the entire period of allergen exposure or it might be administered in a part of that period.

Therefore, in various embodiments of the invention, the unrelated antigen is administered to an individual in need thereof by combined administration of the unrelated antigen to the mucosa of the oral cavity and the administration of the same unrelated antigen or a variant thereof performed simultaneously, contemporaneously, separately or sequentially, in either order.

It is not considered that the unrelated antigen need not be administered to the target organ simultaneous and within the same period of time where the target organ is exposed to the allergen, e.g. to the respiratory tract, gastro-intestinal tract, or to the skin, as the time period may be adjusted to as to achieve bystander suppression. That is to say that the phrase "in a period coinciding with the exposure to" is meant to encompass that the unrelated antigen is administered just before exposure, at the start of exposure, at the end of exposure, within the entire period of exposure, almost within in the period of exposure, and now and then during the exposure. For example, the unrelated antigen is presented to the target organ few hours (1-6 hours), few days (1-4 days) or few (1-5 weeks) or few months (1-5 months) before the allerergen exposure takes place at the target organ. However, preferably, the unrelated antigen is administered to, exposed to or available at the target organ substantially within in the same period as the "triggering" allergen, such as at least simultaneously.

For example, where the allergen-containing source material is an airborne environmental source material, e.g. pollen, mite feces, mite crops, animal dander, animal hair, animal stool, that reaches the respiratory tract, the unrelated antigen might additionally be administered to the respiratory tract, such as to the nasal cavity. For example, a dosing regiment may include the administration of the unrelated antigen to the mucosa of the oral cavity, such as in the form of a sublingual tablet, and the additional administration of the unrelated antigen to the nasal cavity, such as in the form of a nasal spray or nasal drops.

Furthermore, where the allergen-containing source material is a dietary source material, e.g. food or a food-containing product, the unrelated antigen might additionally be administered to the gastro-intestinal tract, such as by ingestion or per-oral administration of a tablet or a capsule.

Likewise, where the allergen-containing source material is an environmental source material, e.g. an environmental source material associated with allergic responses of the skin (e.g. a gum or a gum-containing product), the unrelated antigen might additionally be administered to the skin, such as in the form of a crème, paste, gel or plaster.

It should also be understood that the additional administration of the unrelated antigen to the respiratory tract, gastro-intestinal tract, or skin need not be limited to the period of exposure to the allergen, but may additionally take place out-site the period of exposure to the allergen triggering the hypersensitivity immune response.

It is anticipated that in such embodiments, where the unrelated antigen is administered to the mucosa of the oral cavity as well as to either; the respiratory tract, gastro-intestinal tract or skin, this might be performed simultaneously, contemporaneously, separately or by sequentially administering, in either order: i) a therapeutically effective amount of an unrelated antigen to the oral cavity; and ii) an effective amount of the same unrelated antigen or an variant thereof to either the gastro-intestinal tract, gastro-intestinal tract of skin. At least it might be envisaged that the first step in treatment of or prophylactic treatment of is the administration of the unrelated antigen to the mucosa of the oral cavity. Furthermore, the additional administration of the unrelated antigen to the respiratory tract, gastro-intestinal tract or skin is initiated subsequently, such as days, months or years after starting the administration of unrelated antigen to the oral mucosa. Thus, in some embodiments of the invention, the administration of the unrelated antigen to the oral cavity is initiated before the administration of the unrelated antigen to the respiratory tract, gastro-intestinal tract or skin.

It is also envisaged that the additional administration of the unrelated antigen at least be performed during the individual's exposure to the "triggering" allergen, such as during the pollen season or when ingesting allergen-containing foods. It is also envisaged that the additional administration of the unrelated antigen be continued after the administration of the unrelated antigen to the mucosa of the oral cavity has stopped. Thus, in various embodiments of the invention, the administration of the unrelated antigen to the respiratory tract, gastro-intestinal tract, or skin at least be performed during the individual's exposure to an allergen-containing source material of interest, such as to an allergen to which the individual is sensitized to, such as to an allergen triggering a hypersensitivity immune response in the individual or in a group of individuals of interest; the administration of the unrelated antigen to the respiratory tract, gastro-intestinal tract, or skin is initiated in the period of administering the unrelated antigen to the oral cavity and continued after the administration to the oral cavity has stopped; the administration of the unrelated antigen to the oral cavity is performed daily, twice weekly, weekly or twice a month; and/or the administration of the unrelated antigen to the respiratory tract, gastro-intestinal tract, or skin is performed daily, twice weekly, weekly or twice a month.

In interesting embodiments of the invention, the unrelated antigen is administered to the oral cavity, such as by sublingual administration, such as by sublingual administration of a tablet (including fast-disintegrating tablet, freeze-dried tablet) and the same unrelated antigen or a variant thereof is administered to the nasal cavity in the form of a nasal spray or nasal drops.

*Slightly differences between unrelated antigen for administration to oral cavity versus administration to the respiratory tract, gastro-intestinal tract or skin.* It should also be considered that the unrelated antigen as administered to the respiratory tract, gastro-intestinal tract or skin need not be identical to unrelated antigen administered to the oral cavity. Thus, in some embodiments of the invention, the unrelated antigen that is administered to the respiratory tract, gastro-intestinal tract or skin is a variant of the unrelated antigen of the unrelated antigen administered to the oral cavity or vice versa. However, still preferably, the unrelated antigen used in the step of administering the unrelated antigen to the oral cavity is the same as the unrelated antigen used in the administration of unrelated antigen to the respiratory tract (e.g. nasal cavity), gastro-intestinal tract (e.g. per-oral administration or by ingestion) or to the skin.

However, it is considered that the variant of the unrelated antigen is chemical modified or biologically modified so as to modify its solubility properties, bioavailability properties or stability. Examples are antigen variants with chemical modifications, such as N- or O-linked glycosylation or derivatizing of the N-terminus or of thiol groups of the unrelated antigen. Other examples are antigen variants that are biologically modified, such as by post-translational modifications, such as for example; glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

It should be understood that the variant has significant homology or identity to the original unrelated antigen, such as at least 50 percent, 55 percent, 60 percent, 65 percent, 70 percent, 75 percent, 80 percent, 85 percent, 90 percent, 95 percent, 98 percent and/or 100 percent homology or identity with the amino acid sequence of the unrelated antigen administered to the oral cavity.

Exemplary protein variants may be a substitution variant, deletion variant, duplication variant, insertion variant, insertion-deletion variant or frame shift variant or any other suitable variant.

For example, the variant has no more than 30% of the amino acids of the unrelated antigen as administered to the oral cavity subjected to substitution, deletion, duplication, insertion, or a mixture thereof. More preferably no more than 25%, such as even more preferably no more than 20%, such as no more than 15%, such as no more than 10%, such as no more than 5% of the amino acids of the originally detected unrelated antigen are subjected to substitution, deletion, duplication, insertion, or a mixture thereof. Also it is anticipated that a variant of the unrelated antigen that is administered to the oral cavity has less than 20%, such as less than 15%, less than 10, 8, 6, 5, 4, or 3% amino acids subjected to derivatization, such as glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

### Formulations

Where the antigen exhibits poor stability to the gastric juice, the unrelated antigen is preferably administered in a form avoiding the contact to the gastric juice, such as in a form preventing the degradation of the unrelated antigen in the gastric juice. This may be accomplished by incorporating the unrelated antigen in pharmaceutical formulations resistant to the gastric juice or by incorporating other pharmaceutical delivery techniques able to avoiding the degradation of proteins in the gastric fluid.

The antigen of the invention may be formulated together with therapeutically inactive ingredients and/or immune-modifying agents like adjuvants. Typically, the formulation is a solid dosage form, such as a fast-disintegrating tablet or a liquid including a solution, a suspension, a dispersion, a gelled liquid. Alternatively, the formulation is an emulsion or a re-dissolvable powder, granulate or lyophilisate, which can be dissolved to form a liquid before being administered.

Excipients for use in formulations are well-known to the person skilled in the art and include solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, chitosan, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1 BETA, IL-2, IL-7, IL-12, INFGAMMA), GM-CSF, MDF derivatives, CpG oligonucleotides, LPS, MPL, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl thpeptide, and phospatidylethanolamine.

### List of References

Brimnes et al. Sublingual immunotherapy reduces allergic symptoms in a mouse model of rhinitis. Clinical and experimental allergy, vol 37, no 4, p 488-497, 2007.
Brimnes et al. Sublingual immunotherapy (SLIT) induce systemic tolerance to naïve mice. J Clin Allergy and Clinical Immunology, vol 123, 2, p S127, 2009.
Dahlmann-Hoglund et al. Bystander suppression of the immune response to human serum albumin in rats, Immunology 86, 128-133, 1995.
Dunkin D, Berin MC, Mayer L. Allergic sensitization can be induced via multiple physiologic routes in an adjuvant-dependent manner. J Allergy Clin Immunol 2011.
Jin H, He R, Oyoshi M, Geha RS. Animal models of atopic dermatitis. J Invest Dermatol 2009; 129(1):31-40.
Kildsgaard et al. Sublingual immunotherapy (SLIT) in sensitized mice induces mucosal IgA antibodies. J Clin Allergy and Clinical Immunology, vol 115, 2, p S207, 2005.
Kildsgaard et al. Sublingual immunotherapy in sensitized mice. Annals of allergy, vol 98, 4, p 366-372, 2007.
Miller et al. Antigen-driven Bystander Suppression after Oral Administration of Antigens, J. Exp. Med. 174, 791-798, 1991.
Millington et al. Induction of Bystander Suppression by Feeding Antigen Occurs despite Normal Clonal Expansion of the Bystander T Cell Population. Immunology, 173: 6059-6064, 2004.
Oliveira CR et al. Bystander effect in synergi to anergy in oral tolerance of Blomia Tropcalis/Ovalbumin Murine Co-Immunization model. J Clin Immunol, 25, 153-161, 2005.
Ramos et al. Cell-mediated immune response to unrelated proteins and unspecific inflammation blocked by orally tolerated proteins. Immunology, 126, 354-362, 2008.
Rask et al. Shorter dosing intervals of sublingual immunotherapy leads to more efficacious treatment in a mouse model of allergic inflammation. Scandinavian journal of immunology, vol 71, no 6, p 403-412, 2010.
Sabatos-Peyton C et al. Antigen-specific immunotherapy of autoimmune and allergic diseases. Current opinion in Immunology, 22, 1-7, 2010.
Spergel JM, Mizoguchi E, Oettgen H, Bhan AK, Geha RS. Roles of TH1 and TH2 cytokines in a murine model of allergic dermatitis. J Clin Invest 1999; 103(8):1103-11.
Wang Lifang et al. Antigen driven bystander effect accelerates epicutaneous sensitization with a new protein allergen. J Biomed Science, vol 16, 28, p 1423-0127, 2009.
Weiner et al. Oral tolerance; immune mechanisms and treatment of autoimmune diseases. Immunology of today, vol 18, no 7, p 335-343, 1997.

### Examples

### Example 1

### Prophylactic treatment of naïve mice with an unrelated antigen.

### Methods:

An extract of grass pollen of the species phleum pratense (Phl p) is obtained by extracting defatted grass pollen with an aqueous saline solution.

### Animals

Female, 6-10 week-old BALB/cJ mice were bred in-house and maintained on a defined diet not containing component cross reacting with antisera to Phl p. Each experimental group consisted of 8 animals.

### Animal experiments

Naïve mice received sublingual immunotherapy (SLIT) with 40 µg Phl p extract or buffer for two weeks before being immunized to raise an immune response against another antigen. SLIT was performed by holding the scruff of the mice and carefully applying 5 µl of allergen solution under the tongue. The mice were held by the scruff for additional 20 seconds to prevent the animal from swallowing the allergen solution. The mice were then challenged by intraperitoneal injection of either 8 µg Phl p extract mixed with 250 µg chicken ovalbumin (OVA) adsorbed to aluminium hydroxide or of OVA alone adsorbed to aluminium hydroxide so as to develop an immune response. Ten to twelve days later the mice were euthanized, spleens were removed and cells were set up in an in vitro re-stimulation assay. In this assay, cells were stimulated with the antigen triggering an immune response (OVA) in order to evaluate the effect of SLIT treatment of mice with the unrelated antigens present in the Phl p extract on the development of an OVA specific immune response. In alternative experiments mice were SLIT treated with OVA and intraperitoneally injected with Phl p extract mixed with OVA or Phl p extract alone. Following euthanization, spleen cells were re-stimulated by Phl p to evaluate the effect of the sublingual pre-treatment with OVA on the Phl p specific immune response. The experimental outline is depicted in figure 1.

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37 °C and 5% CO₂. Proliferation was measured by adding 0.5 µCi of 3H-thymidine to each well for the last 18 hours of the culture period, followed by harvesting the cells and counting the incorporated radiolabel.

### Cytokine measurements

Spleens were teased into single cell suspension and washed three times in medium. Cells were counted and adjusted to 3 x10⁶ cells/mL. 5 x 10⁶ cells were added to each well of a 96 well culture plate and the cells were stimulated by 0 and 125 µg/mL OVA. Supernatants were harvested at day 5 and analyzed for the presence of the cytokines IL-4 and IL-5 using a Mesoscale mouse Th1/Th2 ELISA kit.

### Results

### SLIT treatment with Phl p

Figure 2 shows the proliferation rate of spleen cells from mice that have either been SLIT pre-treated with Phl p or with buffer. Following the SLIT treatment, the mice were i.p. immunized with either OVA plus Phl p together (panel A) or OVA alone (panel B) and subsequently spleen cells were re-stimulated in vitro with OVA. The results of Figure 2 indicate that prophylactic SLIT treatment with an antigen (Phl p) unrelated to the antigen triggering the immune response (OVA) down regulates the splenic T-cell response against OVA. However this only occurs when Phl p is present together with OVA at the time of immunization. Also as shown in figure 3, the SLIT pre-treatment with Phl p is capable of down regulating the levels of the cytokines IL-4 and IL-5 in the OVA stimulated T-cell cultures. The cytokines IL-4 and IL-5 are induced by the i.p. immunization with OVA antigen and are cytokines usually induced in Th2-driven immune response.

### SLIT treatment with OVA

Figure 4 shows the proliferation of spleen cells from mice that have been SLIT treated with either OVA or buffer. Following the SLIT treatment the mice were i.p. immunized with either Phl p and OVA together (panel A) or Phl p alone (panel B) and subsequently spleen cells were re-stimulated in vitro with Phl p. The results indicate that prophylactic SLIT treatment with OVA (unrelated antigen) is able to down regulate the splenic T-cell response against Phl p.

Together these data demonstrate that suppression of an immune response (characterized by Th2-cytokines) by SLIT treatment of the mice with an antigen unrelated to the antigen triggering the immune response can be obtained, regardless whether OVA or Phl p extract served as model antigens of the unrelated antigen.

### Example 2

### Comparison of the sublingual route versus the per-oral route of administering an unrelated antigen

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phl p. Each experimental group consisted of 8 animals.

### Animal experiments

Naïve mice were treated daily with 40 µg Phl p extract either by the sublingual or the peroral (intragastric gavache) route for two weeks. The mice were then immunized to raise an immune response against another antigen by intraperitoneal injection. This injection consisted of 8 µg Phl p extract mixed with 250 µg ovalbumin (OVA) adsorbed to aluminium hydroxide. Ten to twelve days later the mice were euthanized, spleens were removed and cells were set up in an in vitro re-stimulation assay. In this assay the cells were stimulated with OVA in order to evaluate the effect of SLIT treatment of with the unrelated antigen Phl p on the OVA specific immune response. The experimental outline is depicted in figure 5.

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37°C and 5% CO2. Proliferation was measured by adding 0.5 µCi of 3H-thymidine to each well for the last 18 hours of the culture period, followed by harvesting the cells and counting the incorporated radiolabel.

### Results:

As seen in figure 6, the OVA-specific proliferation of spleen cells from mice SLIT treated with Phl p prior to the i.p. immunization with Phl p and OVA together was significantly decreased compared to buffer treated mice. This was the case regardless whether the spleen cells were stimulated with a high or low dose of OVA. In contrast, the proliferation of spleen cells from mice per-orally treated with the same dose of Phl p as the SLIT treated mice, was not down regulates to the same extent as observed in the SLIT treated mice. These data shows that SLIT is more effective in suppression an immune response triggered by another antigen compared to per-oral treatment.

### Example 3

### Prophylactic treatment of naive mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Naive mice were treated by sublingual immunotherapy (SLIT) with 40 µg Phl p extract for 2 weeks. Subsequently, the mice were immunized by three weekly i.p. injections of either a mix of 10 µg OVA and 8 µg Phl p extract or 10 µg OVA alone adsorbed to aluminium hydroxide. Subsequently, the mice were challenged intra-nasally (IN) with 50 µg of OVA for four days so as to induce clinically relevant readouts of a Th2-driven immune response. The mice were sacrificed one day after the last challenge and blood, bronchoalveolar fluid (BAL), spleen and cervical lymph nodes were collected for analysis. The experimental outline is depicted in figure 7.

### Evaluating clinically relevant readouts

Clinically relevant readouts, such as airway hyper-reactivity and the fraction of eosinophils, were obtained on the last day of IN challenge.

*Airway hyper-reactivity:* Using a whole body pletysmograph, airflow obstruction was induced by challenging the mice with increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37°C and 5% CO2. Proliferation was measured by adding 0.5 µCi of 3H-thymidine to each well for the last 18 hours of the culture period, followed by harvesting the cells and counting the incorporated radiolabel.

### Results

By these experiments, the inventors wanted to examine whether the prophylactic SLIT treatment also had an effect on allergic asthma. In short, naïve mice were SLIT treated with an unrelated antigen (Phl p) or with buffer prior to the induction of allergic asthma. The asthma was induced by first immunizing the mice by i.p. injection with OVA (antigen triggering the immune response) and Phl p together versus OVA alone, followed by intranasal challenge with the same OVA. As demonstrated by the results depicted in Figure 8, Phl p SLIT treated mice showed reduced airway hyperresponsiveness compared to buffer treated mice (Panel A), whereas this suppression could not be observed in mice that were only immunized with OVA (panel B). Panel C and D shows the percentage of eosinophils in bronchoalveolar lavage (BAL) fluid. Here it can be seen that the fraction of eosinophils was down regulated in mice SLIT treated with an unrelated antigen compared to mice SLIT treated with buffer. Again this suppression could only be observed, when the unrelated antigen (Phl p) was co-administered together with the sensitizing antigen (OVA) at the i.p. immunization. Panels E and F show in-vitro proliferation of cervical lymph node cells. In line with the ability to down-regulate eosinophils in BAL, the OVA-specific in vitro proliferation of cLN cells was down regulated in the Phl p SLIT treated mice, but only when both the sensitizing antigen and the unrelated antigen was present at the time during the i.p. injection.

The data in this series of experiments show that prophylactic SLIT treatment with an unrelated antigen suppresses clinically relevant readouts of allergic asthma in mice.

### Example 4

### Treatment sensitized mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

In one set of experiments mice were sensitized by three weekly i.p. injections of 8 µg Phl p extract adsorbed to aluminium hydroxide. Subsequently, the mice were treated by sublingual immunotherapy (SLIT) with 250 µg OVA for 4 weeks, followed by 2 weeks of intranasal challenge with 8 µg Phl p together with 10 µg OVA or Phl p alone. The experimental outline is depicted in figure 9.

In another set of experiments, mice were sensitized by two weekly i.p. injections of 10 µg OVA adsorbed to aluminium hydroxide. Following this, the mice were SLIT treated with 40 µg Phl p for 4 weeks and subsequently they were intra-nasally challenged for 4 days with 100 µg OVA together with 8 µg Phl p or with 8 µg OVA alone. The experimental outline of this is depicted in figure 13.

In both sets of experiments, the mice were sacrificed one day after the last challenge and blood, bronchoalveolar fluid (BAL), spleen and cervical lymph nodes were collected for analysis.

### Evaluating clinically relevant readouts

Clinically relevant readouts, such as sneezes, airway hyper-reactivity and presence of eosinophils, were obtained on the last day of IN challenge.

*Sneezing:* The mice were observed in an 8 min-period after intranasal administration of Phl p and the numbers of sneezes were counted during this period.

*Airway hyper-reactivity:* Using a whole body pletysmograph, airflow obstruction was induced by increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### Results

### Suppression induced by SLIT treatment with Phl p

In this set of experiments the inventors wanted to investigate if SLIT treatment with one antigen (Phl p) could prevent clinical signs of asthma induced by another antigen (OVA) in a mouse animal model of asthma. As shown in Figure 10 Panel A, the fraction of eosinophils in the BAL fluid was significantly decreased in OVA sensitized mice SLIT treated with Phl p (unrelated antigen) versus mice SLIT treated with buffer, upon the subsequent intranasal challenge with OVA (antigen triggering the immune response) and Phl p together. This down regulation of eosinophils could not be observed if the mice were intra-nasally challenged with OVA alone (Figure 10, Panel B). Furthermore, as seen in figure 11A, the SLIT treatment with Phl p suppressed the IL-5 levels in the BAL fluid compared to SLIT buffer treated mice, provided that the mice were exposed to both OVA and Phl p upon intranasal challenge. This downregulation could not be observed in mice that were IN challenged with OVA alone (Fig 11B). Finally, Figure 12 demonstrates a tendency to the suppression of the airway hyper responsiveness in the Phl p SLIT treated mice. Again, this effect was dependent on the co-exposure of OVA and Phl p during the intranasal challenge.

### Suppression induced by SLIT treatment with OVA

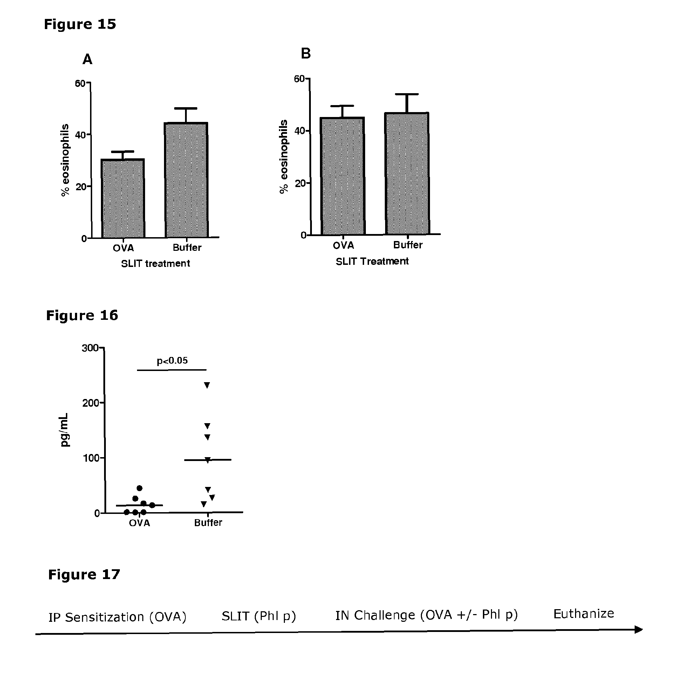

In this set of experiments, the inventors investigated if SLIT treatment with an unrelated antigen (OVA) could prevent clinical and immunological signs of asthma induced by another antigen (phl p) in a mouse animal model of rhinitis. In the rhinitis model employed, the primary clinical read-out is the number of sneezes. As shown in Figure 14, Panel A, Phl p sensitized mice SLIT treated with OVA had significantly less sneezes compared to buffer SLIT treated mice upon the intranasal challenge. This reduction in sneezes was dependent on the presence of both the antigen used for SLIT treatment (OVA) as well as the antigen (Phl p) responsible for inducing allergic rhinitis (Figure 14, Panel B). Furthermore, as observed in Figure 15, Panel A, the fraction of eosinophils in the BAL fluid isolated from OVA SLIT treated mice tend to be reduced, but only if both Phl p and OVA (unrelated antigen) were both presence upon intranasal challenge (Figure 15 Panel B). Finally, as seen in Figure 16, the levels of IL-5 in cell culture supernatants of cLN cells obtained from phl p pre-sensitized mice SLIT treated with OVA were also significantly suppressed upon the intra-nasal challenge with both the sensitizing antigen (Phl p) and OVA (unrelated antigen) together.

In summary, these results show that it is possible to suppress a clinically relevant readout of allergic rhinitis in mice pre-sensitized to an antigen by SLIT treatment of the mice with an unrelated antigen provided the mice are exposed to both the antigen triggering the allergic rhinitis and the unrelated antigen upon challenging respiratory organ of the mice to the triggering antigen.

### Example 5

### Treatment sensitized mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Mice were sensitized by two weekly i.p. injection of 10 µg OVA adsorbed to aluminium hydroxide. Following this, the mice were SLIT treated with 40 µg Phl p for 4 weeks and subsequently they were intra-nasally challenged for 4 days with 50 µg OVA together with 8 µg Phl p or with OVA alone. At the last day of the intranasal challenge, airway hyperresponsiveness was measured using whole body plethysmography. The mice were sacrificed one day after the last challenge and blood, bronchoalveolar fluid (BAL), spleen and cervical lymph nodes were collected for analysis. The experimental outline is depicted in figure 17.

### Evaluating clinically relevant readouts

Clinically relevant readouts, such as airway hyper-reactivity and presence of eosinophils, were obtained on the last day of IN challenge.

*Airway hyper-reactivity:* Using a whole body pletysmograph, airflow obstruction was induced by increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### Results

In this set of experiments, the inventors wanted to confirm the results of Example 4.

As observed Figure 18, Panel A, the airway hyperresponsesiveness was significantly decreased in OVA pre-sensitized mice subsequently SLIT treated with Phl p (unrelated antigen) compared to mice subsequently SLIT treated with buffer prior to the intranasal challenge with OVA (disease-eliciting antigen) and Phl p together. Moreover, a trend towards a decrease in the fraction of eosinophils in the BAL of Phl p SLIT treated mice could be observed (Figure 18, Panel B). Finally, a decrease in the in vitro spleen cell proliferation was observed in phl p SLIT treated mice upon stimulation with OVA (Figure 18C).

Taken together, these results confirm the results of Example 4, and thereby strengthen the observation that an allergic immune response can be suppressed by treatment of pre-sensitized mice with an unrelated antigen.

### Example 6

### Prophylactic treatment of naïve mice with an unrelated antigen so as to reduce a hypersensitivity immune response.

### Methods:

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Naive mice were treated by sublingual immunotherapy (SLIT) with 50 or 200 µg Phl p 5 for 2 weeks, followed by three weekly i.p. injections of either a mix of 10 µg OVA and 10 µg Phl p 5 adsorbed to aluminium hydroxide. Subsequently, the mice were challenged intranasally with 50 µg of OVA for four days. The mice were sacrificed one day after the last challenge and blood, bronchoalveolar fluid (BAL), spleen and cervical lymph nodes were collected for analysis. The experimental outline is depicted in figure 7.

### Clinical data

Clinical data were obtained 24 hours after the last day of IN challenge.

*Differential counting of BAL fluid:* The BAL fluid was centrifuged and the supernatant was removed. The pellet was re-suspended in PBS and the fraction of eosinophils was determined by an automated cell counter (Sysmex).

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37 °C and 5% CO2. Proliferation was measured by adding 0.5 µCi of 3H-thymidine to each well for the last 18 hours of the culture period, followed by harvesting the cells and counting the incorporated radiolabel.

### Results:

In this experiment, the results of example 3 are confirmed using a single antigen for the induction of tolerance by the SLIT treatment instead of the Phl p extract used in example 3. This was done by SLIT treatment of naïve mice with Phl p 5 or buffer. After the SLIT treatment allergic asthma was induced by an IP challenge with OVA and Phl p 5 together or OVA alone, followed by intranasal challenge with OVA. As seen in figure 19A, the fraction of eosinophils in the BAL fluid is reduced in mice SLIT treated with Phl p 5 compared to buffer treated mice, when the mice were subsequently sensitized with OVA together with Phl p and intranasally challenged with OVA. Figure 19B shows the spleen cell proliferation upon in vitro re-stimulation with OVA. In this figure it can be seen that SLIT treatment with Phl p 5 is able to downregulate the OVA-specific in vitro proliferation of spleen cells, when Phl p was present at the time of the IP sensitization.

The data in this experiment show that prophylactic SLIT treatment with a single antigen induces bystander tolerance that has an effect on the clinical read-outs of a subsequently induced allergic asthma.

### Example 7

### Detection of unrelated antigen in an allergen source material

### Methods

A number of antigens (unrelated antigen) that do not bind to sera of allergic patients were detected in pollen allergen extracts or mite allergen extracts by crossed radio immunoelectrophoresis (CIE).

Aqueous extracts of pollen allergens of Phleum Pratense (Phl p) or of mite (Dermatophagoides pteronyssinus (Der p) and Dermatophagoides farinae (Der f) allergens (mite bodies and culture medium) were prepared by the following extracting procedure:
For extraction of whole animals (mite bodies), the mite bodies are grinded in a mortar before extraction. Mite culture and pollen are extracted directly.
Extraction 1:10 are carried out as follows; 1 g of pollen (Phl p) or 1 g of mite bodies + mite culture (Der p or Der f) were suspended in 10 ml of aqueous solution of 0.125 M NH₄HCO₃ and 15 mM NaN₃ and left overnight at +5°C under stirring. The suspension was centrifuged at 27.000 g for 30 minutes at +5°C followed by filtration, if necessary. The precipitate was discarded. The extract was dialysed 3 x 4hrs against 5 mM NH₄HCO₃ followed by 4hrs against purified water. The volume of dialysis water was 20-50 times the volume in the dialysis bag. The dialysed extract is then lyophilised at -80°C and stored in an air tight container at -20°C.

Sera of 21 grass allergic patients and 21 mite (Der p and/or Der f) allergic patients were collected, respectively. Sera of allergy class ≥ 2 were included in the study. Patients had a positive skin prick test and/or presence of IgE antibodies towards the allergens of Phl p and of mites (Der f and Der p), respectively.

Sera containing polyclonal IgG antibodies were produced by immunising rabbits (3-4 months old that never have been immunised before) with the aqueous allergen extracts of Phl p and mites (Der p and Der f), respectively. Sera is collected from coagulated blood and 0.09% NaN₃ is added.

One crossed immunoelectrophoresis is performed for each individual serum by adding the aqueous allergen extract of Phl p or Der p of Der f and respective rabbit antibodies to precipitate the antigens to a CIE plate. The plates were dried in cold air and incubated overnight in small boxes with 300µl patient serum + 7700µl phosphate buffer. The plates were washed 4x10 minutes with 0.9% NaCl and incubated overnight with I¹²⁵ - antiIgE (Pharmacia), 300.000 cpm / plate in a total volume of 8 ml. After washing (4 x 10 min with NaCl) the plates were dried and placed in an X-ray cassette, gel-side up and an X-ray film (Retina) on top. The film was exposed at -80°C for 1 day, 3 days and 10 days. The CIE-plates are stained (Coomassie-staining) and each autoradiography was labeled and cut out.

All precipitates (radioactive + non-radioactive precipitates) are detected, scored and numbered. The radioactively stained precipitates are antigens capable of binding to IgE of patient sera and are then allergens, which may be scored by assigning a score of 3 if a precipitate is visible after 1 day (strong), score 2 if visible after 3 days (moderate) and score 1 if visible after 10 days (weak). Precipitates with no radioactive staining of precipitates are antigens not recognised by IgE antibodies of allergic patients and represents unrelated antigens of the present invention.

An Allergogram is drawn using the precipitate numbers from the reference picture and the number of patients reacting to each precipitate.

### Results

### Phl p:

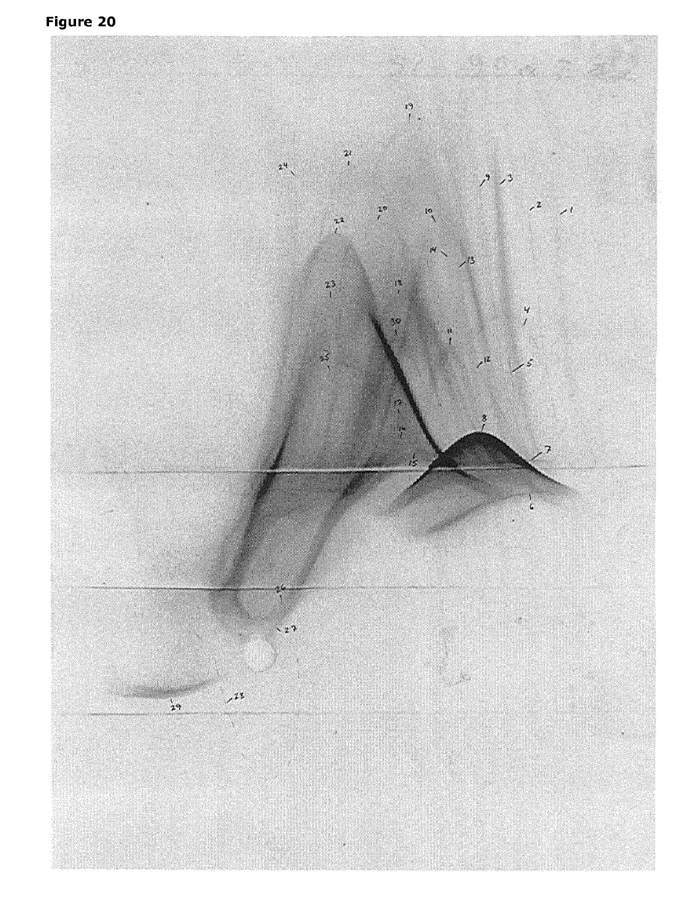

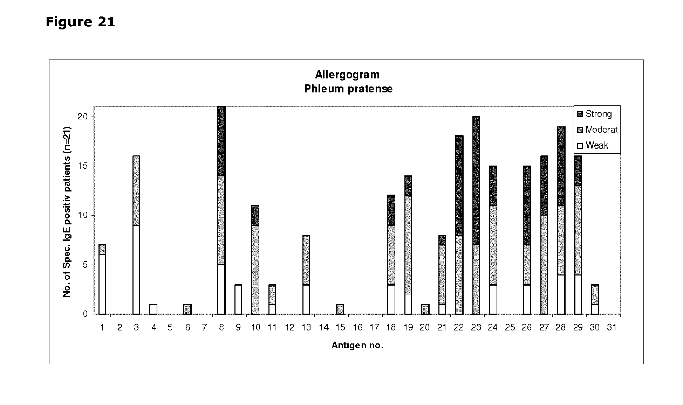

A total of 30 precipitates are visible of which 22 are recognised as allergens and 8 are unrelated antigens of the invention. A CIE diagram (51-990282) is shown in figure 20 and an Allergogram is shown in figure 21. As observed from the Allergogram, the antigen numbered 2, 5, 7, 12, 14, 16, 17 and 31 are non-radioactive precipates and are candidates of an unrelated antigen of the invention.

### Der p:

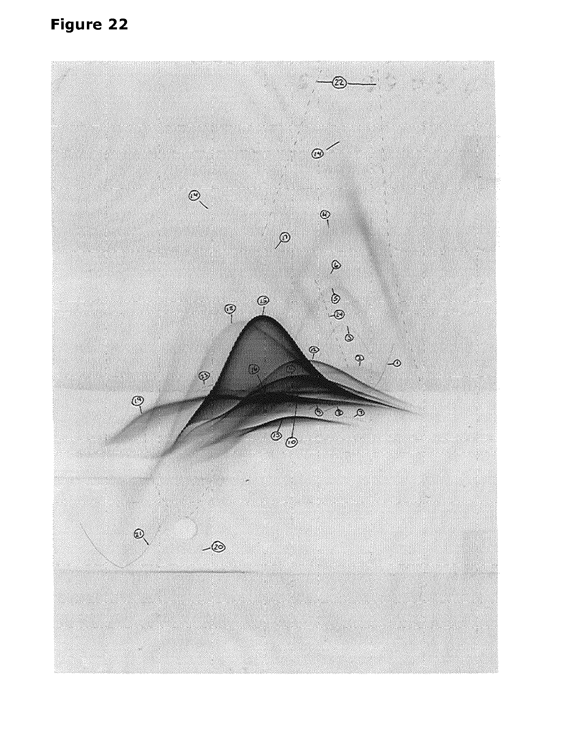

A total of 24 precipitates are visible of which 19 are recognised as allergens and 5 are unrelated antigens of the invention. A CIE diagram is shown in figure 22 and an Allergogram is shown in figure 22. As observed from the Allergogram, the antigen numbered 1, 3, 7, 8, and 9 are non-radioactive precipates and are candidates of an unrelated antigen of the invention.

### Der f:

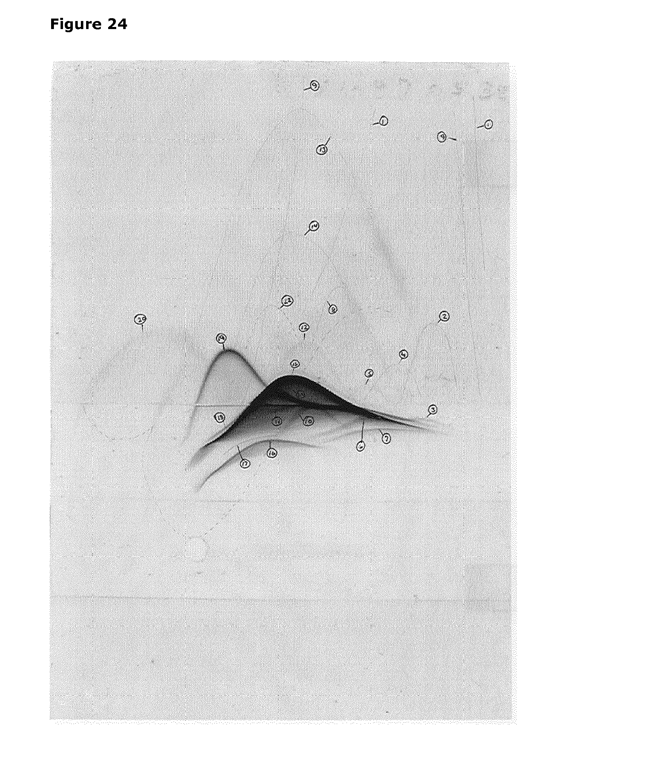

A total of 22 precipitates are visible of which 16 are recognised as allergens and 6 are unrelated antigens of the invention. A CIE diagram is shown in figure 24 and an Allergogram is shown in figure 21. As observed from the Allergogram, the antigen numbered 2, 3, 4, 5, 10 and 17 are non-radioactive precipates and are candidates of an unrelated antigen of the invention.

Thus, unrelated antigens are found in various allergen sources. Further identification of the unrelated antigen may be accomplished by well-known techniques in the art including purifying the precipitate and conducting mass spectrometry analysis.

### Example 8

### Investigating bystander suppression in mice model of Atopic Dermatitis

It will be investigated if SLIT also has a bystander effect in a mouse model of Atopic Dermatitis. For example, allergens obtainable from latex will be used as the "triggering" allergen.

### Methods

Atopic Dermatitis will be induced according to the model developed by Spergel et al (1999) and by Jin H et al (2009). In brief, mice will be sensitized epicutaneously by tape stripping the skin and adminstration of OVA secured by sterile gauze and a bioocclusive dressing. The mice will receive a total of three one week exposures separated by 2 week intervals.

The degree of atopic dermatitis will then be evaluated by obtaining specimens from the patched areas of the skin 24 h after the patch from the third sensitization has been removed.

The specimens will be used for histological analyses to evaluate the thickening of the dermis and epidermis as well as the cellular influx (e.g. eosinophils). Furthermore, the specimens will be stained for cellular markers such as CD3, CD4 and CD8 by immunohistochemistry in order to measure the celluler influx of immune cells. Finally, blood will be collected and cytokines measured using a mesoscale TH1/TH2 ELISA kit.

The effect of SLIT will be investigated in this model by either prophylactic SLIT treatment or by SLIT treatment after the first or second cutaneous administrations of OVA with an antigen non-related to OVA (e.g. Phl p or latex allergen).

### Example 9

### Investigating bystander suppression in mice model of food allergy

It will be investigated if SLIT also has a bystander effect in a mouse model of food allergy.

### Methods:

Food allergy will be induced according to the model described by Dunkin et al (2011). In brief, mice will be epicutaneously sensitized by administration of 0,1 mg OVA in 50 µL on the abdominal skin of anesthetized mice. The sensitization procedure will be repeated weekly for 6 times. One week after the last sensitization the mice will be orally challenged by increasing oral doses of OVA and symptoms of anaphylaxis as well as body temperature will be recorded every 10 minutes.

In addition to the clinical read-outs mentioned above, blood will be collected from the mice following the oral challenge and specific IgE will be measured in serum. Finally, T-cell reactivity in spleen and local draining lymph nodes will also be measured.

The effect of SLIT will be investigated in this model by either prophylactic SLIT treatment prior to the sensitization or by SLIT treatment after the sensitization procedure, with an antigen non-related to OVA (e.g. Phl p or a peanut allergen).

### NUMBERED EMBODIMENTS

Numbered embodiments of the invention are listed below:
Embodiment 1. An antigen for use in treatment or prophylactic treatment of a hypersensitivity immune response in an individual in need thereof, wherein the immune response is triggered by an allergen upon the individual's exposure to a source material comprising said allergen and wherein
   i) the antigen is unrelated to the allergen(s) triggering the hypersensitivity immune response in the individual and is obtainable from or is derivable from said source material; and
   ii) the antigen is administered in a therapeutically effective amount to said individual.
Embodiment 2. The antigen for use according to embodiment 1, wherein when the antigen is for use in treatment of a hypersensitivity immune response in an individual in need thereof the individual has specific IgE antibodies against the allergen(s).
Embodiment 3. The antigen for use according to embodiment 1, wherein when the antigen is for use in prophylactic treatment of a hypersensitivity immune response in an individual in need thereof, the individual does not have specific IgE antibodies against the allergen at the time of initiating the prophylactic treatment.
Embodiment 4. The antigen for use according to embodiment 3, wherein when the antigen is for use in prophylactic treatment of a hypersensitivity immune response in an individual in need thereof, the antigen is unrelated to an allergen of a pre-selected source material comprising an allergen to which a group of individuals have specific IgE antibodies against and is obtainable and/or is derivable from said pre-selected source material.
Embodiment 5. The antigen for use according to any one of embodiments 2 or 3, wherein the individual in need thereof is an individual in risk of being sensitized to an allergen.
Embodiment 6. The antigen for use according to any one of embodiments 1 and 2, wherein the antigen is unrelated to said allergen(s) in the sense that the antigen cannot bind to IgE antibodies obtained from the individual in need of being treated.
Embodiment 7. The antigen for use according to any one of the embodiments 3, 4 and 5, wherein the antigen is unrelated to said allergen(s) in the sense that the antigen cannot bind to serum IgE antibodies obtained from a group of individuals having specific IgE antibodies against said allergen(s).
Embodiment 8. The antigen for use according to any one of the preceding embodiments, wherein the antigen is unrelated to said allergen(s) in the sense that the antigen does not share any B-cell and/or T-cell epitopes with said allergen(s).
Embodiment 9. The antigen for use according to any one of the preceding embodiments, wherein the antigen has an amino acid sequence having less than 70% identity in relation to the amino acid sequence of the allergen(s).
Embodiment 10. The antigen for use according to any one of the preceding embodiments, wherein the antigen does not bind to IgE antibodies obtained from the individual to be treated.
Embodiment 11. The antigen for use according to any one of the preceding embodiments, wherein the antigen is a variant of the originally antigen obtainable or derivable from the source material.
Embodiment 12. The antigen for use according to any one of the preceding embodiments, wherein the source material is an environmental source material or a dietary source material.
Embodiment 13. The antigen for use according to any one of the preceding embodiments, wherein the antigen is obtainable from or is derivable from more than one source material each containing the same unrelated antigen.
Embodiment 14. The antigen for use according to any one of the preceding embodiments, wherein the antigen or a variant thereof is isolated from the source material.
Embodiment 15. The antigen for use according to any one of the preceding embodiments, wherein the antigen or the variant thereof is isolated from another source material comprising the same antigen or a variant thereof.
Embodiment 16. The antigen for use according to any one of the preceding embodiments, wherein the antigen or a variant thereof is reproduced by means of chemically and/or biological methods.
Embodiment 17. The antigen for use according to any one of the preceding embodiments, wherein the antigen variant has an amino acid chain length in the range of 50% to 150% in relation to the originally detected unrelated antigen.
Embodiment 18. The antigen for use according to any one of the preceding embodiments, wherein the antigen variant has at least 50 percent homology in relation to the originally detected unrelated antigen.
Embodiment 19. The antigen for use according to any one of the preceding embodiments, wherein the antigen variant has no more than 30% of the amino acids of the originally detected unrelated antigen subjected to substitution, deletion, duplication, insertion, or a mixture thereof.
Embodiment 20. The antigen for use according to any one of the preceding embodiments, wherein the antigen is a water-soluble antigen.
Embodiment 21. The antigen for use according to any one of the preceding embodiments, wherein the antigen is extractable from the source material in an aqueous solution having pH in the range of 6 to 8.
Embodiment 22. The antigen for use according to any one of the preceding embodiments, wherein the antigen is extractable by subjecting the source material to an aqueous solution having pH in the range of 6 to 8 for a period not exceeding 60 minutes.
Embodiment 23. The antigen for use according to any one of the preceding embodiments, wherein the antigen is co-extractable with the allergen triggering the hypersensitivity immune reaction in the individual by subjecting the source material to an aqueous solution having pH in the range of 6 to 8 for a period not exceeding 60 minutes.
Embodiment 24. The antigen for use according to any one of the preceding embodiments, wherein the antigen is unrelated to a major allergen of the source material.
Embodiment 25. The antigen for use according to any one of the preceding embodiments, wherein the antigen is unrelated to all allergens of the source material.
Embodiment 26. The antigen for use according to any one of the preceding embodiments, wherein the unrelated antigen is an allergen not detected in an allergen-containing environmental source material or an allergen-containing dietary source material.
Embodiment 27. The antigen for use according to any one of the embodiments 1-26 for use in treatment or prophylactic treatment of a hypersensitivity immune response of the respiratory tract in an individual in need thereof, wherein the immune response is triggered by an allergen upon the individual's exposure to an airborne environmental source material comprising said allergen.
Embodiment 28. The antigen for use according to any one of the embodiments 1-26 for use in treatment or prophylactic treatment of a hypersensitivity immune response of the gastro-intestinal tract in an individual in need thereof, wherein the immune response is triggered by an allergen upon the individual's exposure to a dietary source material comprising said allergen.
Embodiment 29. The antigen for use according to any one of the embodiments 1-26 for use in treatment or prophylactic treatment of a hypersensitivity immune response of the skin in an individual in need thereof, wherein the immune response is triggered by an allergen upon the individual's exposure to an environmental source material comprising said allergen.
Embodiment 30. The antigen for use according to any one of the embodiments 1-29 for use in treatment or prophylactic treatment of a hypersensitivity immune response of the respiratory tract in an individual in need thereof, wherein the immune response is triggered by an allergen upon the individual's exposure to an airborne source material comprising said allergen.
Embodiment 31. The antigen for use according to any one of the embodiments 1-30, wherein the antigen is unrelated to a major allergen found in an environmental source material or a dietary source material derived from the major taxonomic groups selected from the group consisting of Animalia Arthropoda, Animalia Chordata, Funghi Ascomycetes, Plantae Corniferopsida, Plantae Liliopsida and Plantae Magnoliopsida.
Embodiment 32. The antigen for use according to any one of the embodiments 1-30, wherein the antigen is unrelated to all allergens found in an environmental source material or a dietary source material derived from the major taxonomic groups selected from the group consisting of Animalia Arthropoda, Animalia Chordata, Funghi Ascomycetes, Plantae Corniferopsida, Plantae Liliopsida and Plantae Magnoliopsida.
Embodiment 33. The antigen for use according to any one of the preceding embodiments 1-30, wherein the antigen is unrelated to a major allergen found in an environmental source material or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales and Proteales.
Embodiment 34. The antigen for use according to any one of the embodiments 1-30, wherein the antigen is unrelated to a all allergens found in an environmental source material or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales and Proteales.
Embodiment 35. The antigen for use according to any one of the embodiments 1-30, wherein the source material is selected from the group consisting of mite crops and mite feces of the major taxonomic group Animalia Arthropoda, dander, hair, saliva, stools or other secretes derived from the major taxonomic group Animalia Chordata; spores or particles derived from the major taxonomic group Funghi Ascomycetes, pollen derived from the major taxonomic group Corniferopsida, pollen derived from the major taxonomic group Plantae Magnoliopsida, pollen derived from the major taxonomic group Plantae Liliopsidae; venoms or secretes derived from the major taxonomic group Animalia Arthropoda; gums or products comprising such a gum derived from trees of the major taxonomic group Plantae Magnoliopsidae.
Embodiment 36. The antigen for use according to any one of the embodiments 1-30, wherein the source material is selected from the group consisting of shrimps or a shrimp-containing food of the major taxonomic group Animalia Arthropoda; lobster or a lobster-containing food of the major taxonomic group Animalia Arthropoda; fruit, legumes, cereals or beans derived from the major taxonomic group Plantae Liliopsidae or a food product comprising such fruits, legumes, cereals and/or beans; fruit, legumes, cereals or beans derived from the major taxonomic group Plantae Magnoliopsidae or a food product comprising such fruits, legumes, cereals and/or beans; nut or a nut-containing food of the from the major taxonomic group Plantae Magnoliopsidae.
Embodiment 37. The antigen for use according to any one of the embodiments 1-30, wherein the source material is selected from the group consisting of cow's milk, cow's milk-containing food, chicken egg white, chicken egg white-containing food, fish or fish-containing food.
Embodiment 38. The antigen for use according to any one of the embodiments 1-30, wherein the source material is a crop and/or feces of a mite selected from the group consisting of mites of the genus Acarus, Glycyphagus, Lepidoglyphus, Tyrophagus, Blomia, Dermatophagoides, Euroglyphus, Blatella and Periplaneta.
Embodiment 39. The antigen for use according to any one of the embodiments 1-30 and 38, wherein the antigen is unrelated to one or more or all of the allergens selected from the group consisting of Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9 and Per a 10
Embodiment 40. The antigen for use according to any one of the embodiments 1-30, wherein the source material is dander, hair, saliva or stools of an animal of the genus canis, felis or Equus.
Embodiment 41. The antigen for use according to any one of the embodiments 1-30 and 40, wherein the antigen is unrelated to one or more or all of the allergens selected from the group consisting of Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5.
Embodiment 42. The antigen for use according to any one of the embodiments 1-30, wherein the source material is yeast, mould or funghi of the genus selected from the group consisting of Cladosporium, Aspergillus, Penicillium, Alternaria and Candida.
Embodiment 43. The antigen for use according to any one of the embodiments 1-30 and 42, wherein the antigen is unrelated to one or more or all of the allergens selected from the group consisting of Cla c 9m Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2.
Embodiment 44. The antigen for use according to any one of the embodiments 1-30, wherein the source material is pollen of the major taxonomic group Corniferopsida, Plantae Liliopsida or Plantae Magnoliopsida.
Embodiment 45. The antigen for use according to any one of the embodiments 1-30 and 44, wherein the source material is pollen of the genus selected from the group consisting of Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea and Platanus.
Embodiment 46. The antigen for use according to any one of the embodiments 1-30 and 44 and 45, wherein the antigen is unrelated to one or more or all of the allergens selected from the group consisting of Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol I 1, Hol I 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1 Zea m 12, Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3.
Embodiment 47. The antigen for use according to any one of the embodiments 1-35, wherein the antigen is for use in treatment or prophylactic treatment of a hypersensitivity immune response of the respiratory tract in an individual in need thereof.
Embodiment 48. The antigen for use according to any one of the embodiments 1-30, wherein the source material is venom of a bee of the genus selected from the group consisting of Apis Bombus, Dolichovespula, Polistes, Polybia, Vespa and Vespula.
Embodiment 49. The antigen for use according to any one of the embodiments 1-30 and 48, wherein the antigen is unrelated to one or more of the allergens selected from the group consisting of Api c 1, Api d 1; Api m 1, Api m 2, Api m 3, Api m 4, Api m 5, Api m 6, Api m 7, Api m 8, Api m 9, Api m 10, Api m 11, Bom p 1, Bom p 4; Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5; Pol g 1, Pol g 5, Poly p 1, Poly s 5; Vesp c 1, Vesp c 5, Vesp ma 2, Vesp ma 5, Vesp m 1, Vesp m 5, Ves g 5, Ves m 1, Ves m 2, Ves m 5; Ves p 5; Ves s 1, Ves s 5; Ves vi 5; Ves v 1, Ves v 2, Ves v 3 and Ves v 5.
Embodiment 50. The antigen for use according to any one of the embodiments 1-30 and 48 and 49, wherein the antigen is for use in treatment or prophylactic treatment of a hypersensitivity immune response of the skin and/or anaphylaxis. Embodiment 51. The antigen for use according to any one of the embodiments 1-30, wherein the source material is a gum or products comprising such a gum derived from the genus Hevea.
Embodiment 52. The antigen for use according to any one of the embodiments 1-30 and 51, wherein the antigen is unrelated to one or more of the allergens selected from the group consisting of Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14.
Embodiment 53. The antigen for use according to any one of the embodiments 1-30 and 51 to 52, wherein the antigen is for use in treatment or prophylactic treatment of a hypersensitivity immune response of the skin.
Embodiment 53. The antigen for use according to any one of the embodiments 1-30, wherein source material is a dietary source material derived from a genus selected from the group consisting of Hordeum, Oryza, Secale, Triticum, Zea, Arachis, Corylis, Juglans, Prunus, Anacardium, Pistacia and Glycine.
Embodiment 54. The antigen for use according to any one of the embodiments 1-30 and 53, wherein the antigen is unrelated to one or more of the allergens selected from the group consisting of Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and Gly m 6.
Embodiment 55. The antigen for use according to any one of the embodiments 1-30, wherein source material is a dietary source material derived from the group consisting of cow's milk, milk-containing food, chicken egg white, chicken egg white-containing food, fish or fish-containing food.
Embodiment 56. The antigen for use according to any one of the embodiments 1-30 and 55, wherein the antigen is unrelated to one or more of the allergens selected from the group consisting ofcasein of cow's milk, beta-lacto-globulin of cow's milk, ovalbumin of chicken egg white, ovomucoid of chicken egg white and allergen M of a fish.
Embodiment 57. The antigen for use according to any one of the embodiments 53 to 56, wherein dietary source material is a food ingredient or a product comprising such a food ingredient.
Embodiment 58. The antigen for use according to any one of the preceding embodiments, wherein the hypersensitivity immune response is associated with an allergic immune response.
Embodiment 59. The antigen for use according to any one of the preceding embodiments, wherein the hypersensitivity immune response is a type 1 hypersensitivity immune response.
Embodiment 60. The antigen for use according to any one of the preceding embodiments, wherein the hypersensitivity immune response is associated with atopic dermatitis, urticaria, contact dermatitis, allergic conjunctivitis, allergic rhinitis, allergic asthma, anapylaxis, food allergy and/or drug allergy.
Embodiment 61. The antigen for use according to any one of the preceding embodiments, wherein the antigen is administered to a mucosa of the oral cavity.
Embodiment 62. The antigen for use according to any one of the preceding embodiments, wherein the antigen is administered to the sublingual mucosa.
Embodiment 63. The antigen for use according to any one of the preceding embodiments, wherein the administration to the oral cavity does not comprise subsequent swallowing of the antigen.
Embodiment 64. The antigen for use according to any one of the preceding embodiments, wherein an allergen is not co-administered together with the unrelated antigen.
Embodiment 65. The antigen for use according to any one of the preceding embodiments, wherein when the source material is an airborne environmental source material, the unrelated antigen is also administered to the respiratory tract within a period at least coinciding partly or entirely with the individual's exposure to said source material.
Embodiment 66. The antigen for use according to any one of the preceding embodiments, wherein when the source material is an airborne environmental source material, the unrelated antigen is also administered to the nasal cavity within a period at least coinciding partly or entirely with the individual's exposure to said source material.
Embodiment 67. The antigen for use according to any one of the preceding embodiments, wherein when the allergen-containing source material is a dietary source material, the unrelated antigen is also administered to the gastro-intestinal tract within a period at least coinciding partly or entirely with the individual's exposure to said source material.
Embodiment 68. The antigen for use according to any one of the preceding embodiments, wherein when the allergen-containing source material is a dietary source material, the unrelated antigen is also ingested or administered per-orally within a period at least coinciding partly or entirely with the individual's exposure to said source material.
Embodiment 69. The antigen for use according to any one of the preceding embodiments, wherein when the allergen-containing source is a gum or a gum-containing product, the unrelated antigen is also administered to the skin within a period at least coinciding partly or entirely with the individual's exposure to said source material.
Embodiment 70. The antigen for use according to any one of the preceding embodiments, wherein the combined administration of the unrelated antigen to the mucosa of the oral cavity and the administration of the same unrelated antigen or a variant thereof is performed simultaneously, contemporaneously, separately or sequentially, in either order.
Embodiment 71. The antigen for use according to any one of the preceding embodiments, wherein the unrelated antigen that is administered to the respiratory tract, gastro-intestinal tract or skin is a variant of the unrelated antigen of the unrelated antigen administered to the oral cavity or vice versa.
Embodiment 72. The antigen for use according to any one of the preceding embodiments, wherein the administration of the unrelated antigen to the respiratory tract, gastro-intestinal tract, or skin is performed out-site the period of exposure to the allergen triggering the hypersensitivity immune response.
Embodiment 73. The antigen for use according to any one of the preceding embodiments, wherein the administration of the unrelated antigen to the oral cavity is initiated before the administration of the unrelated antigen to the respiratory tract, gastro-intestinal tract or skin.
Embodiment 74. The antigen for use according to any one of the preceding embodiments, wherein the administration of the unrelated antigen to the respiratory tract, gastro-intestinal tract, or skin at least be performed during the individual's exposure to an allergen triggering the hypersensitivity immune response in the individual.
Embodiment 75. The antigen for use according to any one of the preceding embodiments, wherein the administration of the unrelated antigen to the respiratory tract, gastro-intestinal tract, or skin is initiated in the period of administering the unrelated antigen to the oral cavity and continued after the administration to the oral cavity has stopped.
Embodiment 76. The antigen for use according to any one of the preceding embodiments, wherein the administration of the unrelated antigen to the oral cavity is performed daily, twice weekly, weekly or twice a month.
Embodiment 77. The antigen for use according to any one of the preceding embodiments, wherein the administration of the unrelated antigen to the respiratory tract, gastro-intestinal tract, or skin is performed daily, twice weekly, weekly or twice a month.
Embodiment 78. A method for treatment or prophylactic treatment of a hypersensitivity immune response in an individual in need thereof, such as to a method for bystander suppression of a hypersensitivity immune response in an individual in need thereof, wherein the immune response is triggered by an allergen upon the individual's exposure to a source material comprising said allergen and wherein;
   i) the antigen is unrelated to the allergen(s) triggering the hypersensitivity immune response in the individual and is obtainable from or is derivable from said source material; and
   ii) the antigen is administered in a therapeutically effective amount to said individual.
Embodiment 79. Use of an antigen for the manufacturing of a medicament for use in treatment or prophylactic treatment of a hypersensitivity immune response in an individual in need thereof, such as to the use of an antigen for the manufacturing of a medicament for use in bystander suppression of a hypersensitivity immune response in an individual in need thereof, wherein the immune response is triggered by an allergen upon the individual's exposure to a source material comprising said allergen and wherein
   i) the antigen is unrelated to the allergen(s) triggering the hypersensitivity immune response in the individual and is obtainable from or is derivable from said source material; and
   ii) the antigen is administered in a therapeutically effective amount to said individual.

## Claims

1. A protein antigen for use in treatment or prophylactic treatment of a hypersensitivity immune response in an individual in need thereof, wherein the immune response is triggered by an allergen upon the individual's exposure to a source material comprising said allergen and wherein
i) the antigen is unrelated to the allergen(s) triggering the hypersensitivity immune response in the individual and is obtainable from or is derivable from said source material; and
ii) the protein antigen is administered in a therapeutically effective amount to said individual without being co-administered together with the allergen.

2. The protein antigen for use according to any claim 1, wherein the antigen is unrelated to said allergen(s)
- in the sense that the antigen cannot bind to IgE antibodies obtained from the individual in need of being treated, or
- in the sense that the antigen cannot bind to serum IgE antibodies obtained from a group of individuals having specific IgE antibodies against said allergen(s), or
- in the sense that the antigen does not share any B-cell and/or T-cell epitopes with said allergen(s).

3. The protein antigen for use according to claim 1 or 2, wherein the antigen has an amino acid sequence having less than 70% identity in relation to the amino acid sequence of the allergen(s).

4. The protein antigen for use according to any one of the preceding claims, wherein the antigen is a variant of the originally antigen obtainable or derivable from the source material.

5. The protein antigen for use according to any one of the preceding claims, wherein the source material is an environmental source material or a dietary source material.

6. The protein antigen for use according to any one of the preceding claims, wherein the antigen variant
- has an amino acid chain length in the range of 50% to 150% in relation to the originally detected unrelated antigen and/or
- has at least 50 percent homology in relation to the originally detected unrelated antigen, and/or
- has no more than 30% of the amino acids of the originally detected unrelated antigen subjected to substitution, deletion, duplication, insertion, or a mixture thereof.

7. The protein antigen for use according to any one of the preceding claims, wherein the antigen is a water-soluble antigen.

8. The protein antigen for use according to any one of the preceding claims, wherein
- the antigen is extractable from the source material in an aqueous solution having pH in the range of 6 to 8, preferably by subjecting the source material to an aqueous solution having pH in the range of 6 to 8 for a period not exceeding 60 minutes and/or
- the antigen is co-extractable with the allergen triggering the hypersensitivity immune reaction in the individual by subjecting the source material to an aqueous solution having pH in the range of 6 to 8 for a period not exceeding 60 minutes.

9. The protein antigen for use according to any one of the preceding claims, wherein the antigen is unrelated to a major allergen of the source material, and preferably unrelated to all allergens of the source material.

10. The protein antigen for use according to any one of the preceding claims for use in treatment or prophylactic treatment of
- a hypersensitivity immune response of the respiratory tract in an individual in need thereof, wherein the immune response is triggered by an allergen upon the individual's exposure to an airborne environmental source material comprising said allergen, or
- a hypersensitivity immune response of the gastro-intestinal tract in an individual in need thereof, wherein the immune response is triggered by an allergen upon the individual's exposure to a dietary source material comprising said allergen, or
- a hypersensitivity immune response of the skin in an individual in need thereof, wherein the immune response is triggered by an allergen upon the individual's exposure to an environmental source material comprising said allergen, or
- a hypersensitivity immune response of the respiratory tract in an individual in need thereof, wherein the immune response is triggered by an allergen upon the individual's exposure to an airborne source material comprising said allergen.

11. The antigen for use according to any one of the claims 1-30, wherein the antigen is unrelated to
- a major allergen found in an environmental source material or a dietary source material derived from the major taxonomic groups selected from the group consisting of Animalia Arthropoda, Animalia Chordata, Funghi Ascomycetes, Plantae Corniferopsida, Plantae Liliopsida, and Plantae Magnoliopsida, or
- all allergens found in an environmental source material or a dietary source material derived from the major taxonomic groups selected from the group consisting of Animalia Arthropoda, Animalia Chordata, Funghi Ascomycetes, Plantae Corniferopsida,Plantae Liliopsida, and Plantae Magnoliopsida, or
- a major allergen found in an environmental source material or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales, and Proteales, or
- all allergens found in an environmental source material or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales, and Proteales.

12. The antigen for use according to any one of the preceding claims, wherein the source material is selected from the group consisting of
- the group consisting of mite crops and mite feces of the major taxonomic group Animalia Arthropoda, dander, hair, saliva, stools or other secretes derived from the major taxonomic group Animalia Chordata; spores or particles derived from the major taxonomic group Funghi Ascomycetes, pollen derived from the major taxonomic group Corniferopsida, pollen derived from the major taxonomic group Plantae Magnoliopsida, pollen derived from the major taxonomic group Plantae Liliopsidae; venoms or secretes derived from the major taxonomic group Animalia Arthropoda; gums or products comprising such a gum derived from trees of the major taxonomic group Plantae Magnoliopsidae; or
- the group consisting of shrimps or a shrimp-containing food of the major taxonomic group Animalia Arthropoda; lobster or a lobster-containing food of the major taxonomic group Animalia Arthropoda; fruit, legumes, cereals or beans derived from the major taxonomic group Plantae Liliopsidae or a food product comprising such fruits, legumes, cereals and/or beans; fruit, legumes, cereals or beans derived from the major taxonomic group Plantae Magnoliopsidae or a food product comprising such fruits, legumes, cereals and/or beans; nut or a nut-containing food of the from the major taxonomic group Plantae Magnoliopsidae; or
- the group consisting of cow's milk, cow's milk-containing food, chicken egg white, chicken egg white-containing food, fish or fish-containing food.

13. The antigen for use according to any one of the preceding claims, wherein the antigen is for use in
- treatment or prophylactic treatment of a hypersensitivity immune response of the respiratory tract in an individual in need thereof, or
- treatment or prophylactic treatment of a hypersensitivity immune response of the skin and/or anaphylaxis, or
- treatment or prophylactic treatment of a hypersensitivity immune response of the skin.

14. The antigen for use according to any one of the preceding claims, wherein the antigen is administered to a mucosa of the oral cavity, such as to the sublingual mucosa, preferably withoug comprising subsequent swallowing of the antigen.

15. The antigen for use according to any one of the preceding claims, wherein the unrelated antigen that is administered to the respiratory tract, gastro-intestinal tract or skin is a variant of the unrelated antigen of the unrelated antigen administered to the oral cavity or vice versa.
